# EUROPEAN PATENT APPLICATION

(11) **EP 2 615 169 A1**
(43) Date of publication of application: **17.07.2013**
(21) Application number: 11818224.5
(22) Date of filing: 18.08.2011
(51) Int. Cl.: C12N 15/09, A01H 5/00, C12N 1/15, C12N 1/19, C12N 1/21, C12N 5/10, C12N 9/02, C12P 17/06, C12Q 1/68

(54) **FLAVIN ENZYME HAVING FLAVONOL 8-HYDROXYLASE ACTIVITY AND USE THEREOF**

(30) Priority: 19.08.2010 JP 2010183875
(71) Applicant: Suntory Holdings Limited, Kita-ku Osaka-shi Osaka 530-8203 (JP)
(72) Inventor: SUZUKI Hideyuki, Kisarazu-shi Chiba 292-0818 (JP)
(74) Representative: Vossius & Partner
(86) International application number: PCT/JP2011/068683
(87) International publication number: WO 2012/023583

(57) **Abstract**

The purpose of the present invention is to provide a novel flavonol 8-hydroxylase. The present invention relates to a flavin enzyme protein having a flavonol 8-hydroxylase activity, and a polynucleotide etc. encoding the same, and so on. The present invention provides: a polynucleotide comprising the nucleotide sequence of SEQ ID NO: 1 or 3; a polynucleotide encoding a protein consisting of the amino acid sequence of SEQ ID No. 2; an expression vector and transformant comprising the polynucleotide; a method for screening a plant which blooms one or more yellow coloured flowers, by using the polynucleotide; a method for producing a plant which blooms one or more yellow coloured flowers by introducing the polynucleotide into host cells; and a method for producing a flavin enzyme protein having a flavonol 8-hydroxylase activity, using the transformant.

### [Sequence listing]

SEQUENCE LISTING

<110>  SUNTORY HOLDINGS LIMITED

<120>  FLAVIN ENZYME HAVING FLAVONOL 8-HYDROXYLASE ACTIVITY AND USE OF
       THE SAME

<130>  PCT11-0041

<150>  JP 2010-183875
<151>  2010-08-19

<160>  6

<170>  PatentIn version 3.4

<210>  1
<211>  1203
<212>  DNA
<213>  Lotus japonicus


<220>
<221>  CDS
<222>  (1)..(1203)

<400>  1
atg gag aga aat gaa gat gtg gag att gtg atc gtg gga ggt ggt ata        48
Met Glu Arg Asn Glu Asp Val Glu Ile Val Ile Val Gly Gly Gly Ile
1               5                   10                  15

tgt ggc ctt gca aca gcc ctc gcc ctt cac agg aag agc att aaa agt        96
Cys Gly Leu Ala Thr Ala Leu Ala Leu His Arg Lys Ser Ile Lys Ser
            20                  25                  30

ttg gtg tta gaa aga tca gac aag ttg cga gct aca gga gca gcc att       144
Leu Val Leu Glu Arg Ser Asp Lys Leu Arg Ala Thr Gly Ala Ala Ile
        35                  40                  45

att gtg cag gca aat ggg tgg cgt gct ctt gat cag cta ggt gtt ggt       192
Ile Val Gln Ala Asn Gly Trp Arg Ala Leu Asp Gln Leu Gly Val Gly
    50                  55                  60

tca att ctt aga caa acg gcc att cca att aaa ggg ggg aaa ttt ata       240
Ser Ile Leu Arg Gln Thr Ala Ile Pro Ile Lys Gly Gly Lys Phe Ile
65                  70                  75                  80

acg ctt aat gag gct gag tca aag gaa ata cca ttt ggg gtt cct cac       288
Thr Leu Asn Glu Ala Glu Ser Lys Glu Ile Pro Phe Gly Val Pro His
                85                  90                  95

gaa ttt cgt tgt ttg aag cgt act gat ctg atg gaa gcc atg gcc aat       336
Glu Phe Arg Cys Leu Lys Arg Thr Asp Leu Met Glu Ala Met Ala Asn
            100                 105                 110

agt tta ccg gag gga act ata cgt acc agt tgt caa gta ctt tcc gtt       384
Ser Leu Pro Glu Gly Thr Ile Arg Thr Ser Cys Gln Val Leu Ser Val
        115                 120                 125

gaa ttg gat cca gta aca cat gat caa aaa ctt ttg cta agc aat gga       432
Glu Leu Asp Pro Val Thr His Asp Gln Lys Leu Leu Leu Ser Asn Gly
    130                 135                 140

acc atc ctt cat gcc aat gtt gtt att gga tgt gat ggc gtg aat act 480
Thr Ile Leu His Ala Asn Val Val Ile Gly Cys Asp Gly Val Asn Thr
145                 150                 155                 160

tgc att gca aac atg gtt gga tta caa cgg tct aat ttc ttg ccc ttc 528
Cys Ile Ala Asn Met Val Gly Leu Gln Arg Ser Asn Phe Leu Pro Phe
                165                 170                 175

ttg aca tgc gtg gcg cga ggc ttc aca aat tat cca aat ggt cat caa 576
Leu Thr Cys Val Ala Arg Gly Phe Thr Asn Tyr Pro Asn Gly His Gln
            180                 185                 190

ttt ggt agt gag ttt gtt atg atg gtc aga ggt cat gtc caa ctt gga 624
Phe Gly Ser Glu Phe Val Met Met Val Arg Gly His Val Gln Leu Gly
        195                 200                 205

aca atc cca gtg acg gaa aag ctt gtt tac tgg ttt tta aca agg cca 672
Thr Ile Pro Val Thr Glu Lys Leu Val Tyr Trp Phe Leu Thr Arg Pro
    210                 215                 220

aaa act tct caa gat tca ata att tca aat aac aca agt ctt att aga 720
Lys Thr Ser Gln Asp Ser Ile Ile Ser Asn Asn Thr Ser Leu Ile Arg
225                 230                 235                 240

caa tct ttg atg gaa tca atg aag ggt ttc cca aca cac gca atg gat 768
Gln Ser Leu Met Glu Ser Met Lys Gly Phe Pro Thr His Ala Met Asp
                245                 250                 255

atg ata caa aat tgc atg ttg aac ttc ttg cat ctc act gac ttg aag 816
Met Ile Gln Asn Cys Met Leu Asn Phe Leu His Leu Thr Asp Leu Lys
            260                 265                 270

tat cgt cca cca tgg gac ttg att ctt agc cac ttc cgg aaa ggc aca 864
Tyr Arg Pro Pro Trp Asp Leu Ile Leu Ser His Phe Arg Lys Gly Thr
        275                 280                 285

ata acc att gct ggc gat gca ctg cat gct aca ggc cca ttc att gca 912
Ile Thr Ile Ala Gly Asp Ala Leu His Ala Thr Gly Pro Phe Ile Ala
    290                 295                 300

caa ggt ggc tca gct tcc ata gaa gat gca att gtt ctt gct aga tgc 960
Gln Gly Gly Ser Ala Ser Ile Glu Asp Ala Ile Val Leu Ala Arg Cys
305                 310                 315                 320

ttg gcc caa aag atg cat agt gct aag aac aac gcg ata aga aag aga 1008
Leu Ala Gln Lys Met His Ser Ala Lys Asn Asn Ala Ile Arg Lys Arg
                325                 330                 335

gat gta gag gaa gca ttt cat caa tat gct aaa gaa agg aag atg aga 1056
Asp Val Glu Glu Ala Phe His Gln Tyr Ala Lys Glu Arg Lys Met Arg
            340                 345                 350

atc ttt tgg atc tct ttg cat agt ttt ctt att ggg aaa aag ctt gat 1104
Ile Phe Trp Ile Ser Leu His Ser Phe Leu Ile Gly Lys Lys Leu Asp
        355                 360                 365

gca caa tca ttc ata atg aaa tcg att atc att gca ctc atg gtg att 1152
Ala Gln Ser Phe Ile Met Lys Ser Ile Ile Ile Ala Leu Met Val Ile
    370                 375                 380

ctg ttt aga gac cca gat tgg cat acc cgc tat gat tgt ggg act cta 1200
Leu Phe Arg Asp Pro Asp Trp His Thr Arg Tyr Asp Cys Gly Thr Leu
385                 390                 395                 400

tag                                                                 1203

<210>  2
<211>  400
<212>  PRT
<213>  Lotus japonicus

<400>  2

Met Glu Arg Asn Glu Asp Val Glu Ile Val Ile Val Gly Gly Gly Ile
1               5                   10                  15

Cys Gly Leu Ala Thr Ala Leu Ala Leu His Arg Lys Ser Ile Lys Ser
            20                  25                  30

Leu Val Leu Glu Arg Ser Asp Lys Leu Arg Ala Thr Gly Ala Ala Ile
        35                  40                  45

Ile Val Gln Ala Asn Gly Trp Arg Ala Leu Asp Gln Leu Gly Val Gly
    50                  55                  60

Ser Ile Leu Arg Gln Thr Ala Ile Pro Ile Lys Gly Gly Lys Phe Ile
65                  70                  75                  80

Thr Leu Asn Glu Ala Glu Ser Lys Glu Ile Pro Phe Gly Val Pro His
                85                  90                  95

Glu Phe Arg Cys Leu Lys Arg Thr Asp Leu Met Glu Ala Met Ala Asn
            100                 105                 110

Ser Leu Pro Glu Gly Thr Ile Arg Thr Ser Cys Gln Val Leu Ser Val
        115                 120                 125

Glu Leu Asp Pro Val Thr His Asp Gln Lys Leu Leu Leu Ser Asn Gly
    130                 135                 140

Thr Ile Leu His Ala Asn Val Val Ile Gly Cys Asp Gly Val Asn Thr
145                 150                 155                 160

Cys Ile Ala Asn Met Val Gly Leu Gln Arg Ser Asn Phe Leu Pro Phe
                165                 170                 175

Leu Thr Cys Val Ala Arg Gly Phe Thr Asn Tyr Pro Asn Gly His Gln
            180                 185                 190

Phe Gly Ser Glu Phe Val Met Met Val Arg Gly His Val Gln Leu Gly
        195                 200                 205

Thr Ile Pro Val Thr Glu Lys Leu Val Tyr Trp Phe Leu Thr Arg Pro
    210                 215                 220

Lys Thr Ser Gln Asp Ser Ile Ile Ser Asn Asn Thr Ser Leu Ile Arg
225                 230                 235                 240

Gln Ser Leu Met Glu Ser Met Lys Gly Phe Pro Thr His Ala Met Asp
                245                 250                 255

Met Ile Gln Asn Cys Met Leu Asn Phe Leu His Leu Thr Asp Leu Lys
            260                 265                 270

Tyr Arg Pro Pro Trp Asp Leu Ile Leu Ser His Phe Arg Lys Gly Thr
        275                 280                 285

Ile Thr Ile Ala Gly Asp Ala Leu His Ala Thr Gly Pro Phe Ile Ala
    290                 295                 300

Gln Gly Gly Ser Ala Ser Ile Glu Asp Ala Ile Val Leu Ala Arg Cys
305                 310                 315                 320

Leu Ala Gln Lys Met His Ser Ala Lys Asn Asn Ala Ile Arg Lys Arg
                325                 330                 335

Asp Val Glu Glu Ala Phe His Gln Tyr Ala Lys Glu Arg Lys Met Arg
            340                 345                 350

Ile Phe Trp Ile Ser Leu His Ser Phe Leu Ile Gly Lys Lys Leu Asp
        355                 360                 365

Ala Gln Ser Phe Ile Met Lys Ser Ile Ile Ile Ala Leu Met Val Ile
    370                 375                 380

Leu Phe Arg Asp Pro Asp Trp His Thr Arg Tyr Asp Cys Gly Thr Leu
385                 390                 395                 400

<210>  3
<211>  1898
<212>  DNA
<213>  Lotus japonicus

<400>  3
atggagagaa atgaagatgt ggagattgtg atcgtgggag gtggtatatg tggccttgca      60

acagccctcg cccttcacag gtttaatatt gcaagccaaa tgttttgttg tttccttctt     120

ctatatcatc atttgattta tatgcatgat ttaatgtttc tttaatttaa gatttcattt     180

gcattaactt aaatgtagga agagcattaa aagtttggtg ttagaaagat cagacaagtt     240

gcgagctaca ggagcagcca ttattgtgca ggcaaatggg tggcgtgctc ttgatcagct     300

aggtgttggt tcaattctta gacaaacggc cattccaatt aaagggtaac atcatctttc 360

gatctcgatc tcgatctcga tgtcgatctc actcactaaa ctaattaatt tgtattggat 420

taactaatat ttagatagct catgaatgat aataacttaa gcatgttgtc agggggaaat 480

ttataacgct taatgaggct gagtcaaagg aaataccatt tgggtgagtt gcatttgcag 540

tgcatttttc aatactatta tgtttaaatt acgtaaatag agcattttac acatcctaat 600

taattttgtt gataaacagg gttcctcacg aatttcgttg tttgaagcgt actgatctga 660

tggaagccat ggccaatagt ttaccggagg gaactatacg taccagttgt caagtacttt 720

ccgttgaatt ggatccagta acacatgatc aaaaactttt gctaagcaat ggaaccatcc 780

ttcatgccaa tgtatatatc tttgtctttt gtacattttc atattttgaa atcttatata 840

ttggtgtcaa aacatatcca cagagtcaat gcctttgttt gcataggtag gggtgaaaat 900

gctagcttgt taaatcttct tagcaagtac tacatttatg aaattgcaat actacaatta 960

cttacttata catttgctgg tgtaaggttg ttattggatg tgatggcgtg aatacttgca 1020

ttgcaaacat ggttggatta caacggtcta atttcttgcc cttcttgaca tgcgtggcgc 1080

gaggcttcac aaattatcca aatggtcatc aatttggtag tgagtttgtt atgatggtca 1140

gaggtcatgt ccaacttgga acaatcccag tgacggaaaa gcttgtttac tggtttttaa 1200

caaggccaaa aacttctcaa ggtcattcgt taactttatc aactttcaat tcaaacaata 1260

cctgctctta tttattttat ttctttggtt aataagttta tcttcaatat atcttttatg 1320

tgttagagat ttaaattttg aggattgatt tttgtatttt tgtttcaacc tacaaagcag 1380

attcaataat ttcaaataac acaagtctta ttagacaatc tttgatggaa tcaatgaagg 1440

gtttcccaac acacgcaatg gatatgatac aaaattgcat gttgaacttc ttgcatctca 1500

ctgacttgaa gtatcgtcca ccatgggact tgattcttag ccacttccgg aaaggcacaa 1560

taaccattgc tggcgatgca ctgcatgcta caggcccatt cattgcacaa ggtggctcag 1620

cttccataga agatgcaatt gttcttgcta gatgcttggc ccaaaagatg catagtgcta 1680

agaacaacgc gataagaaag agagatgtag aggaagcatt tcatcaatat gctaaagaaa 1740

ggaagatgag aatcttttgg atctctttgc atagttttct tattgggaaa aagcttgatg 1800

cacaatcatt cataatgaaa tcgattatca ttgcactcat ggtgattctg tttagagacc 1860

cagattggca tacccgctat gattgtggga ctctatag 1898

<210> 4
<211> 28
<212> DNA
<213> Artificial

<220>
<223> Synthetic DNA

<400>  4
aagaaaatgg agagaaatga agatgtgg                                          28


<210>  5
<211>  26
<212>  DNA
<213>  Artificial

<220>
<223>  Synthetic DNA

<400>  5
ctatagagtc ccacaatcat agcggg                                            26


<210>  6
<211>  1008
<212>  DNA
<213>  Rosa hybrid cultivar

<400>  6
atgggggtag agagagttca agacattgct tctgcaactt ccaaggacac aatcccagtg      60

gagttcatca ggtcagagaa tgagcagcct ggaatcacca ccgtccccgg cacagtcctc     120

gagtgcccta tcattgattt cagcgaccct gatgaggaga aactcctcaa acaaatcttc     180

gaggccagca ccgactgggg catgtaccaa atcgtgaacc acgacatttc taacgaggcc     240

attgccaagt tgcaggccgt cggaaaagag ttctttgagc ttccgcacga ggagaaagag     300

gtttacgcaa aagatccgaa ctctaagtcc gtggagggat acggaacatt tttacagaag     360

gaactagaag ggaagaaagg gtgggtggat catctgttcc ataagatttg gccaccttct     420

gccattaact actgcttttg gcctaagaat ccagcttctt acagggaagc taatgaagag     480

tatgcaaaga atctacacaa ggtggtggag aagctattta aactgttatc tttggggtta     540

gggcttgaag cacaagaact gaagaaggca gttggtggtg atgacttggt gtaccttctc     600

aaaattaatt actacccgcc ttgtccccgc cctgatcttg ctcttggtgt ggttgcccat     660

acggacatgt ccgccctcac cattctcgtc ccaaacgacg ttcagggcct ccaagcttgc     720

cgagatggcc agtggtacga tgtcaaatat atccctaatg ccctagtcat ccacattggt     780

gatcaaatgg aggtaatgag caatggaaag ttcaaggctg tgctgcacag aaccacagtg     840

agcaaagaca agacgagaat ctcttggcca gtgtttctgg aacctccacc agaccatata     900

atagggcctc accccaagct cgtcaatgat aaggagaatc caccaaagta caagaccaag     960

aagtacagcg agtatgtcta caacaagctc aacaagatcc cccagtaa                 1008

## Description

### TECHNICAL FIELD

The present invention relates to a polynucleotide encoding a flavin enzyme protein having a flavonol 8-hydroxylase activity and a method for use thereof.

### BACKGROUND ART

Flavonols including quercetagetin (6-hydroxyquercetin) and gossypetin (8-hydroxyquercetin) exhibit yellow colours because positions 6 and 8 are substituted with hydroxyl group, respectively (Non-Patent Document 1). Almost all plant species produce flavonols but there are only known a very few plant species that manage to produce flavonols, in which position 6 or 8 is substituted with hydroxyl group.

When compared to the yellow colour exhibited by carotenoids or other pigments, the yellow colour exhibited by flavonols has a quiet tone of colour to create an elegant atmosphere and has been heralded among plant viewers. In wild plant species, however, plant species which bloom yellow coloured flowers where flavonols serve as pigments are limited.

In addition, flavonols draw attention not only as pigments but also in view of usefulness as medicaments. For instance, gossypin (gossypetin-8-glucoside) in which sugar is attached to position 8 of gossypetin that is one of flavonols is reportedly a substance which performs free radical scavenging to exhibit an antitumor and anticarcinogenic activity (Non-Patent Document 2).

There are some reports on hydroxylation at position 6 of flavonols. Anzelotti and Ibrahim reported that flavonol 6-hydroxylase is involved in the 6-hydroxylation of partially methylated flavonols from the leaves of Chrysosplenium americanum (Non-Patent Document 3). On the other hand, Halbwirth et al. reports that flavonol 6-hydroxylase involved in hydroxylation at position 6 in petals of Tagetes patula is classified as cytochrome P450-dependent monooxygenase (Non-Patent Document 4). Furthermore, Latunde-Data et al. suggests that cytochrome P-450-dependent flavonoid 6-hydroxylases isolated from elicitor-treated soybean would be specifically involved in biosynthesis of 6-hydroxyisoflavones (Non-Patent Document 5).

On the other hand, hydroxylation at position 8 of flavonols has rarely been reported up to now. In Chrysanthemum segetum, it is only reported by Halbwirth and Stich that a flavin enzyme is involved in hydroxylation at position 6 of quercetin (Non-Patent Document 6).

### [Patent Document]

[Patent Document 1] WO 2006/126294

### [Non-Patent Documents]

[Non-Patent Document 1] Harborne J.B., 1967. Comparative Biochemistry of the Flavonoids. Academic Press, London.
[Non-Patent Document 2] Kunnumakkara A.B. et al., 2007. Blood 109, 5112-5121
[Non-Patent Document 3] Anzellotti D. and Ibrahim R.K., 2004. BMC Plant Biol. 4,20.
[Non-Patent Document 4] Halbwirth H. et al., 2004. Plant Sci. 167, 129-135.
[Non-Patent Document 5] Latunde-Data A.O. et al, 2001. J. Biol. Chem. 276, 1688-1695.
[Non-Patent Document 6] Halbwirth H. and Stich K., 2006. Phytochemistry 67, 1080-1087.

### DISCLOSURE. OF THE INVENTION

### Problems to be Solved by the Invention

Under the foregoing circumstances, it has been expected to isolate a novel flavin enzyme having a flavonol 8-hydroxylase activity and a gene therefor and develop a novel plant species which bloom yellow coloured flowers using the enzyme or gene, whereby flavonols serve as pigments.

### Means to Solve the Problems

The present inventors conducted exhaustive analysis of a group of genes from Lotus japonicus of leguminous plants that are highly expressed in buds, not in flowering time, and further searched genes coding for the proteins using as a coenzyme flavin adenine dinucleotide (FAD) based on information of the steric structures (secondary structures) of putative proteins, not of the putative amino acid sequences (primary structures) of proteins encoded by candidate genes. As a result of extensive studies, the inventors have succeeded in cloning genes encoding flavin enzymes having the flavonol 8-hydroxylase activity. The present invention has thus been accomplished. More specifically, the present invention provides a polynucleotide, protein, expression vector and transformant defined below, a method for screening a plant which blooms one or more yellow coloured flowers using the polynucleotide, a method for producing a plant which blooms one or more yellow coloured flowers by introducing the polynucleotide into host cells, and a method for producing a flavin enzyme having the flavonol 8-hydroxylase activity, using the transformant.

That is, the present invention provides the following features.
[1] A polynucleotide according to any one selected from the group consisting of (a) to (e) below:
   (a) a polynucleotide comprising the nucleotide sequence of SEQ ID NO: 1 or 3;
   (b) a polynucleotide encoding a protein consisting of the amino acid sequence of SEQ ID NO: 2;
   (c) a polynucleotide encoding a flavin enzyme protein consisting of an amino acid sequence wherein 1 to 100 amino acids are deleted, substituted, inserted and/or added in the amino acid sequence of SEQ ID NO: 2, and having a flavonol 8-hydroxylase activity;
   (d) a polynucleotide encoding a flavin enzyme protein having an amino acid sequence having at least 70% identity with the amino acid sequence of SEQ ID NO: 2, and having a flavonols 8-hydroxylase activity; and,
   (e) a polynucleotide that hybridizes to a polynucleotide consisting of a nucleotide sequence complementary to the nucleotide sequence of SEQ ID NO: 1 or 3 under stringent conditions, and that encodes a flavin enzyme protein having a flavonol 8-hydroxylase activity.
[2] The polynucleotide according to [1] above, which is either one defined in (f) or (g) below:
   (f) a polynucleotide encoding a flavin enzyme protein consisting of an amino acid sequence wherein 1 to 10 amino acids are deleted, substituted, inserted and/or added in the amino acid sequence of SEQ ID NO: 2, and having flavonol 8-hydroxylase activity; and,
   (g) a polynucleotide encoding a flavin enzyme protein having an amino acid sequence having at least 90% identity with the amino acid sequence of SEQ ID NO: 2, and having a flavonol 8-hydroxylase activity.
[3] The polynucleotide according to [1] above, comprising the nucleotide sequence of SEQ ID NO: 1 or 3.
[4] The polynucleotide according to [1] above, encoding a protein consisting of the amino acid sequence of SEQ ID NO: 2.
[5] The polynucleotide according to any one of [1] to [4] above, which is a DNA.
[6] A protein encoded by the polynucleotide according to any one of [1] to [5] above.
[7] A vector comprising the polynucleotide according to any one of [1] to [5] above.
[8] A non-human transformants, into which the polynucleotide according to any one of [1] to [5] above is introduced.
[9] A non-human transformant, into which the vector according to [7] above is introduced.
[10] The non-human transformant according to [8] or [9] above, which is a plant.
[11] A method for screening a plant which blooms one or more yellow coloured flowers, which comprises the steps of:
   (1) extracting a polynucleotide from a subject plant;
   (2) hybridizing the polynucleotide to a polynucleotide that hybridizes to a polynucleotide consisting of a nucleotide sequence complementary to the nucleotide sequence of SEQ ID NO: 1 or 3 under stringent conditions; and,
   (3) detecting the hybridization.
[12] A method for producing a plant which blooms one or more yellow coloured flowers, said method comprising introducing the polynucleotide according to any one of [1] to [5] above into a host plant or part thereof.
[13] A method for producing a flavin enzyme protein having a flavonol 8-hydroxylase activity, said method comprising culturing the non-human transformant according to [8] or [9] above.
[14] A processed product of the plant according to [10] above or part thereof.
[15] A method for producing a 8-hydroxylated flavonol, said method comprising reacting the protein according to [6] above or a fragment of the protein having a flavonol 8-hydroxylase activity with a flavonol.

### Effects of the Invention

The polynucleotide of the present invention can be used to transform various host cells (e.g., plant cells). The transformants thus obtained can be used to produce a flavin enzyme protein having the flavonol 8-hydroxylase activity, which is encoded by the polynucleotide of the present invention. The 8-hydroxylated flavonols can also be produced by reacting the protein of the present invention with flavonols.

The plant obtained by transforming plant cells with the polynucleotide of the present invention can bloom yellow coloured flowers, which colour cannot be produced in the wild type, particularly when plant species used as a host fails to bloom yellow coloured flowers in the wild type. Thus, such a plant is worthy of admiration and expected to have very high commercial value. Further according to the screening method of the present invention, plant species which bloom yellow coloured flowers wherein flavonols serve as pigments can be screened by screening the genome of a subject plant seed.

### BRIEF DESCRIPTION OF DRAWINGS

FIG. 1A shows the genome gene sequence of the flavin enzyme of the invention having the flavonol 8-hydroxylase activity.
FIG. 1B shows the genome gene sequence of the flavin enzyme of the invention having the flavonol 8-hydroxylase activity, which is continued from FIG. 1A.
FIG. 2 shows the results of UPLC-TOF MS analysis of the components of the reaction product obtained by reacting the protein of the invention with a flavonol substrate (quercetin).
FIG. 3 shows the results obtained by comparing the peaks identified using mass spectrometry of the reaction products obtained by reacting the protein of the present invention with a flavonol substrate (quercetin), gossypetin, quercetagetin and negative control.
FIG. 4 shows the results of ion trap MS/MS analysis of the components of the reaction product obtained by reacting the protein of the present invention with a flavonol substrate (quercetin).
FIG. 5 shows that the absorption peak (372.00 nm: FIG. 5A) of quercetin is shifted to a different absorption wavelength (384.00 nm: FIG. 5C) by substitution of position 8 in quercetin with hydroxyl group.
FIG. 6 shows the results of UPLC-TOF MS analysis of the components of the reaction product obtained by reacting the protein of the present invention with a flavanone substrate (eriodictyol).
FIG. 7 shows the results of ion trap MS/MS analysis of the components of the reaction product obtained by reacting the protein of the present invention with a flavanone substrate (eriodictyol).
FIG. 8 shows that the absorption peak (288.00 nm: FIG. 8A) of eriodictyol is shifted to a different absorption wavelength (292.00 nm: FIG. 8B) by substitution of position 8 in eriodictyol with hydroxyl group.
FIG. 9 shows the results of UPLC-TOF MS analysis of the components of the reaction product obtained by reacting the protein of the present invention with a flavone substrate (luteolin).
FIG. 10 shows the results of ion trap MS/MS analysis of the components of the reaction product obtained by reacting the protein of the present invention with a flavone substrate (luteolin).
FIG. 11 shows the results of detection of 8-hydroxylated kaempferol synthesized by the transgenic plant of the present invention, using high performance liquid chromatography.
FIG. 12 shows the absorption spectra of 8-hydroxylated kaempferol synthesized by the transgenic plant of the present invention.

### MODE FOR CARRYING OUT THE INVENTION

Hereinafter the present invention is described in detail. The embodiment below is intended to be only by way of example to describe the invention but not limited to this embodiment only. The present invention may be implemented in various ways without departing from the gist of the invention.

All of the publications, published patent applications, patents and other patent documents cited in this application are herein incorporated by reference in their entirety. This application hereby incorporates by reference the contents of the specification and drawings in Japanese Patent Application (No. 2010-183875) filed August 19, 2010, from which the priority was claimed.

The present inventors have succeeded for the first time in cloning the full-length cDNA of flavonol 8-hydroxylase gene derived from Lotus japonicus, as will be later described in detail in EXAMPLES below. The present inventors have also identified the nucleotide sequence of genomic gene of flavonol 8-hydroxylase from Lotus japonicus and the putative amino acid sequence of flavonol 8-hydroxylase encoded by the gene. The CDS sequence, the putative amino acid sequence and the genome sequence of flavonol 8-hydroxylase derived from Lotus japonicus are SEQ ID NO: 1, SEQ ID NO: 2 and SEQ ID NO: 3, respectively. These polynucleotides and enzymes may be obtained by the methods described in EXAMPLES below, known genetic engineering techniques, known methods for synthesis, and so on.

### 1. Polynucleotide of the Invention

First, the present invention provides the polynucleotide described in any one selected from the group consisting of (a) to (e) below:
(a) a polynucleotide comprising the nucleotide sequence of SEQ ID NO: 1 or 3;
(b) a polynucleotide encoding a protein consisting of the amino acid sequence of SEQ ID NO: 2;
(c) a polynucleotide encoding a flavin enzyme protein consisting of an amino acid sequence wherein 1 to 100 amino acids are deleted, substituted, inserted and/or added in the amino acid sequence of SEQ ID NO: 2, and having the flavonol 8-hydroxylase activity;
(d) a polynucleotide encoding a flavin enzyme protein having then amino acid sequence having at least 70% identity with the amino acid sequence of (SEQ ID NO: 2, and having the flavonol 8-hydroxylase activity; and,
(e) a polynucleotide that hybridizes to a polynucleotide consisting of a nucleotide sequence complementary to the nucleotide sequence of SEQ ID NO: 1 or 3 under stringent conditions, and that encodes a flavin enzyme protein having the flavonol 8-hydroxylase activity.

As used herein, the term "polynucleotide" is intended to mean a DNA or RNA.

As used herein, the term "polynucleotide that hybridizes under stringent conditions" refers to, e.g., a polynucleotide consisting of a nucleotide sequence complementary to the nucleotide sequence of SEQ ID NO: 1 or 3, or a polynucleotide obtained by colony hybridization, plaque hybridization, Southern hybridization or the like, using as a probe the whole or part of a polynucleotide consisting of the nucleotide sequence encoding the amino acid sequence of SEQ ID NO: 2. For the methods of hybridization, there may be used the methods described in, e.g., "Sambrook & Russell, Molecular Cloning; A Laboratory Manual Vol. 3, Cold Spring Harbor, Laboratory Press 2001," "Ausubel, Current Protocols in Molecular Biology, John Wiley & Sons 1987-1997," etc.

As used herein, the term "stringent conditions" may be any of low stringent conditions, moderate stringent conditions or high stringent conditions. The term "low stringent conditions" are, for example, 5x SSC, 5x Denhardt's solution, 0.5% SDS and 50% formamide at 32°C. The term "moderate stringent conditions" are, for example, 5x SSC, 5x Denhardt's solution, 0.5% SDS and 50% formamide at 42°C, or 5x SSC, 1% SDS, 50 mM Tris-HCl (pH 7.5) and 50% formamide at 42°C. The term "high stringent conditions" are, for example, 5x SSC, 5x Denhardt's solution, 0.5% SDS and 50% formamide at 50°C or 0.2 x SSC and 0.1% SDS at 65°C. Under these conditions, a DNA with higher identity is expected to be obtained efficiently at higher temperatures, although multiple factors are involved in hybridization stringency including temperature, probe concentration, probe length, ionic strength, time, salt concentration and others, and those ordinarily skilled in the art may appropriately choose these factors to achieve similar stringency.

When commercially available kits are used for hybridization, for example, an Alkphos Direct Labeling and Detection System (GE Healthcare) may be used. In this case, after cultivation with a labeled probe overnight, the membrane is washed with a primary wash buffer containing 0.1% (w/v) SDS at 55°C to detect the hybridized DNA, according to the attached protocol. Alternatively, in producing a probe based on the nucleotide sequence complementary to the nucleotide sequence of SEQ ID NO: 1 or 3 or on the whole or part of the nucleotide sequence encoding the amino acid sequence of SEQ ID NO: 2, hybridization can be detected with a DIG Nucleic Acid Detection Kit (Roche Diagnostics) when the probe is labeled with digoxygenin (DIG) using a reagent commercially available (e.g., a PCR Labeling Mix (Roche Diagnostics), etc.).

In addition to those described above, other polynucleotides that can be hybridized include DNAs having 60% or higher, 61% or higher, 62% or higher, 63% or higher, 64% or higher, 65% or higher, 66% or higher, 67% or higher, 68% or higher, 69% or higher, 70% or higher, 71% or higher, 72% or higher, 73% or higher, 74% or higher, 75% or higher, 76% or higher, 77% or higher, 78% or higher, 79% or higher, 80% or higher, 81 % or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity with the DNA of SEQ ID NO: 1 or 3, or the DNA encoding the amino acid sequence of SEQ ID NO: 2, as calculated by homology search software, such as FASTA and BLAST using default parameters.

Identity between the amino acid sequences or nucleotide sequences may be determined using algorithm BLAST (Basic Local Alignment Search Tool) by Karlin and Altschul (Proc. Natl. Acad. Sci. USA, 87: 2264-2268, 1990; Proc. Nail Acad. Sci. USA, 90: 5873, 1993). Programs called blastn, blastx, blastp, tblastn and tblastx based on the BLAST algorithm have been developed (Altschul S. F. et al., J. Mol. Biol. 215; 403, 1990). When a nucleotide sequence is sequenced using blastn, the parameters are, for example, score=100 and wordlength=12. When an amino acid sequence is sequenced using blastp, the parameters are, for example, score=50 and wordlength=3. When BLAST and Gapped BLAST programs are used, default parameters for each of the programs are employed.

The polynucleotides of the present invention described above can be acquired by known genetic engineering techniques, known methods for synthesis, and so on.

### 2. Protein of the Invention

The present invention provides the proteins shown below.
(i) A protein encoded by the polynucleotide of any one of (a) to (e) above.
(ii) A protein comprising the amino acid sequence of SEQ ID NO: 2.
(iii) A flavin enzyme protein comprising an amino acid sequence wherein one or more amino acids are deleted, substituted, inserted and/or added in the amino acid sequence of SEQ ID NO: 2, and having the flavonol 8-hydroxylase activity.
(iv) A flavin enzyme protein having an amino acid sequence having at least 95% identity with the amino acid sequence of SEQ ID NO: 2, and having the flavonol 8-hydroxylase activity.

The proteins described in (iii) or (iv) above are typically naturally occurring mutants of the protein of SEQ ID NO: 2 and include those proteins which may be artificially obtained using site-directed mutagenesis described in, e.g., "Sambrook & Russell, Molecular Cloning: A Laboratory Manual, Viol. 3, Cold Spring Harbor Laboratory Press 2001," "Ausubel, Current Protocols in Molecular Biology, John Wiley & Sons 1987-1997," "Nuc. Acids. Res., 10, 6487 (1982)," "Proc. Natl. Acad. Sci. USA, 79, 6409 (1982)," "Gene, 34, 315 (1985)," "Nuc. Acids. Res., 13, 4431 (1985)," "Proc. Natl. Acad. Sci. USA, 82, 488 (1985)," etc.

As used herein, "the flavin enzyme protein consisting of an amino acid sequence wherein one or more amino acids are deleted, substituted, inserted and/or added in the amino acid sequence of SEQ ID NO: 2, and having the flavonol 8-hydroxylase activity" includes flavin enzyme proteins consisting of an amino acid sequence wherein, e.g., 1 to 100, 1 to 90, 1 to 80, 1 to 70, 1 to 60, 1 to 50, 1 to 40, 1 to 39, 1 to 38, 1 to 37, 1 to 36, 1 to 35, 1 to 34, 1 to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 7, 1 to 16, 1 to 15, 1 to 14, 1 to 13, 1 to 12, 1 to 11, 1 to 10, 1 to 9 (1 to several), 1 to 8, 1 to 7, 1 to 6, 1 to 5, 1 to 4, 1 to 3, 1 to 2, or one amino acid is/are deleted, substituted, inserted and/or added in the amino acid sequence of SEQ ID NO: 2, and having the flavonol 8-hydroxylase activity. In general, the number of deletions, substitutions, insertions, and/or additions is preferably smaller.

Such proteins include a flavin enzyme protein having an amino acid sequence having the identity of approximately 70% or higher, 75% or higher, 80% or higher, 8 1 % or higher, 82% or higher, 83% or higher, 84% or higher, 85% or higher, 86% or higher, 87% or higher, 88% or higher, 89% or higher, 90% or higher, 91 % or higher, 92% or higher, 93% or higher, 94% or higher, 95% or higher, 96% or higher, 97% or higher, 98% or higher, 99% or higher, 99.1% or higher, 99.2% or higher, 99'3% or higher, 99.4% or higher, 99.5% or higher, 99.6% or higher, 99.7% or higher, 99.8% or higher, or 99.9% or higher, with the amino acid sequence of SEQ ID NO: 2, and having the flavonol 8-hydroxylase activity. As the identity percentage described above is higher, the protein is preferable in general.

As used herein, the term "flavonol 8-hydroxylase activity" is intended to mean an activity that substitutes hydrogen bound to position 8 of the target flavonol molecule with hydroxyl group.

The flavonol 8-hydroxylase activity can be confirmed by transforming appropriate host cells with the polynucleotide of the present invention, collecting crude enzyme from the transformants, reacting the crude enzyme with an appropriate flavonol compound (e.g., quercetin, etc.) and analyzing the composition of the reaction product by known analytical methods, including high performance liquid chromatography (HPLC), gas chromatography, time-of-flight mass spectrometry (TOF-MS), ultra (high) performance liquid chromatography (UPLC), etc, to detect a hydroxide of the flavonol compound. Alternatively, the flavonol 8-hydroxylase activity can be detected by measuring absorbance of a flavonol compound before and after the reaction. The host cells above are not particularly limited and include yeast or cells such as Escherichia coli, etc., which do not contain molecules having the flavonol 8-hydroxylase activity in the natural state. The examples of methods for confirming the flavonol 8-hydroxylase activity specifically includes the following but is not limited thereto:

### (i) Method using yeast

A polynucleotide of interest is introduced into an appropriate yeast expression vector (e.g., pYES2 (Invitrogen)). The resulting yeast expression vector plasmid is introduced into an appropriate yeast strain (e.g., BJ2168 (Nippon Gene)). The resulting transformed yeast is cultured in a suitable selection medium for 12 hours or more and preferably 14 hours or more. Subsequently, the yeast is recovered. Next, the yeast is cultured in a suitable expression medium for 12 hours or more, preferably 24 hours or more, and more preferably, 48 hours or more. The incubation is carried out under atmospheric pressure at 25°C to 37°C, and preferably at 30°C both in the case using the selection medium or expression medium.

Next, the yeast cells are collected and subjected to cell lysis. The cell lysis can be performed using glass beads, a homogenizer, a sonicator, etc. The cell lysate is centrifuged and the supernatant is recovered. The crude enzyme solution of a protein encoded by the polynucleotide to be tested can thus be prepared.

Then, the crude enzyme solution, NADPH, substrate flavonol and a riboflavin, preferably, flavin mononucleotide (FMN), flavin adenine dinucleotide (FAD) or the like, are mixed and reacted at 25°C to 37°C for 15 minutes to 5 hours.

The resulting reaction product is analyzed for its components by HPLC, gas chromatography, etc., by which it can be confirmed if hydroxyl group is attached to the carbon at position 8 of the substrate flavonol. When hydroxyl group is attached to the carbon at position 8 of the substrate flavonol, it is confirmed that the protein encoded by the polynucleotide to be tested has the flavonol 8-hydroxylase activity.

For the details of the method for detecting the flavonol 8-hydroxylase activity using yeast, reference can be made to the description of Mizutani M et al (WO 00/44907).

### (ii) Method using isotope-labeled flavonol substrate

The crude enzyme solution of a protein encoded by the polynucleotide to be tested is prepared in a manner similar to the method described in "(i) method using yeast" above.

The resulting crude enzyme solution, KH₂PO₄/K₂HPO₄ solution, radioisotope-labeled flavonol substrate (e.g., [¹⁴C]-labeled quercetin), FAD and NADPH are mixed and reacted at 25°C to 37°C for 15 minutes to 5 hours. The reaction is stopped by adding acetic acid. Acetic acid is further added to the reaction product to extract the phenol compound. The organic layer is analyzed on a cellulose plate by thin-layer chromatography (TLC). The cellulose plate used for the TLC analysis is scanned (TLC-Linear Analyzer (Berthold, Wildbad, Germany), etc. can be used) to detect and quantify the radioactivity. Thus, it can be confirmed if hydroxyl group is attached to the carbon at position 8 of flavonol substrate labeled with radioisotope.

For the details of the method for detecting the flavonol 8-hydroxylase activity using radioisotope-labeled flavonol substrate, reference can be made to the description of Halbwirth H and Stich K (Photochemistry 67 (2006) 1080-1087).

### (iii) Method by measurement of absorbance

The flavonol compound has the property that the absorption wavelength is shifted to a longer wavelength side by approximately 6 to 10 nm, when position 8 is substituted with hydroxyl group. Consequently, the flavonol 8-hydroxylase activity can be assayed by using this property.

Specifically, the absorption wavelength of the flavonol compound wherein position 8 of substrate flavonol is substituted with hydroxyl group (hereinafter "positive control absorption wavelength") is first measured.

Next, a crude enzyme solution is prepared in a manner similar to the method described in "(i) Method using yeast" above and the absorption wavelength of the crude enzyme (hereinafter, "negative control absorption wavelength") is measured.

Then, the crude enzyme above is mixed with the flavonol substrate to react them and the absorption wavelength of the reaction product is measured.

When the absorption wavelength peak shifted to a longer wavelength side by approximately 6 to 10 nm than the peak of the negative control absorption wavelength (hereinafter "longer wavelength-shifted absorption peak") is detected from the reaction product and the wavelength of the longer wavelength-shifted absorption peak matches the positive control absorption wavelength, it means that the 8-hydroxylated flavonol compound is contained in the reaction product. It can be determined in this case that the crude enzyme described above has the flavonol 8-hydroxylase activity.

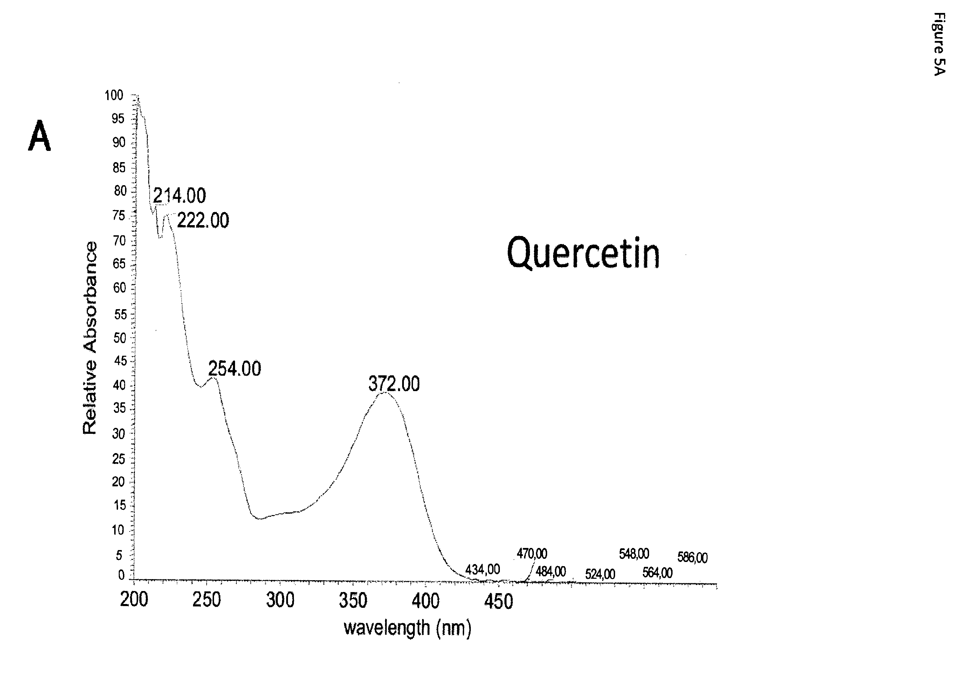

It is conformed, for example, in EXAMPLES that the absorption wavelength of quercetin as substrate flavonol was shifted from 372 nm to 384 nm after the reaction with the protein of the present invention (FIG. 5A - FIG. 5C).

In the present invention, the term "flavonol" is not particularly limited so long as it is the compound represented by general formula (I) below.

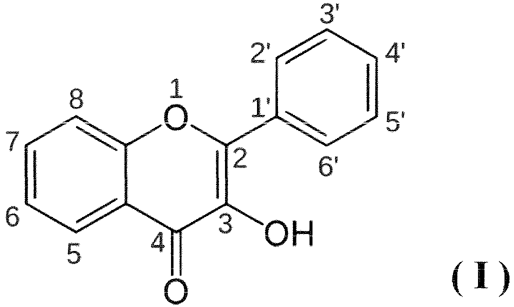

Examples of flavonols which serve as substrate (hereinafter, "substrate flavonol") include, but not limited to, flavonol compounds such as 3-hydroxyflavone, azaleatin, fisetin, galangin, kaempferide, kaempferol, isorhamnetin, morin, myricetin, pachypodol, quercetin, rhamnazin, rhamnetin, and the like. Alternatively, the substrate may also be derivatives of the flavonol compounds described above e.g., glycosides of the flavonol compounds described above, so long as position 8 can be substituted with hydroxyl group. The substrate for the protein of the present invention may further include flavones such as luteolin, apigenin, etc., flavanones such as eriodictyol, naringenin, etc., dihydroflavonols such as dihydroquercetin, etc., which have skeletons similar to the flavonol.

Preferred examples of the substrate flavonol are flavonol compounds that produce a yellow colour when hydroxyl group is bound to position 8.

Specific examples of the flavonol compounds that produce a stronger yellow colour when hydroxyl group is bound at position 8 than that of intact state include quercetin, kaempferol, myricetin, etc. For example, when quercetin is used as the substrate, the position 8 is substituted with hydroxyl group to form gossypetin.

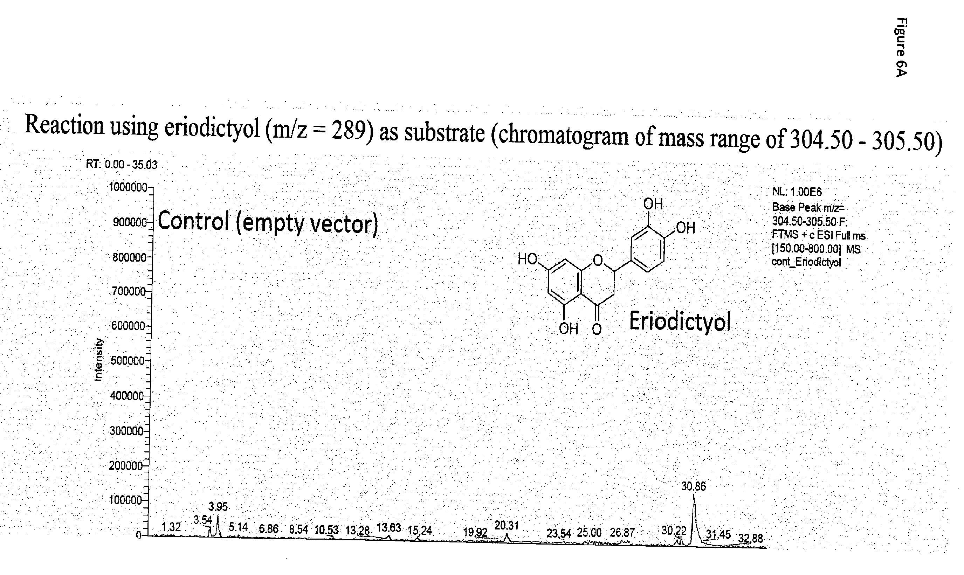

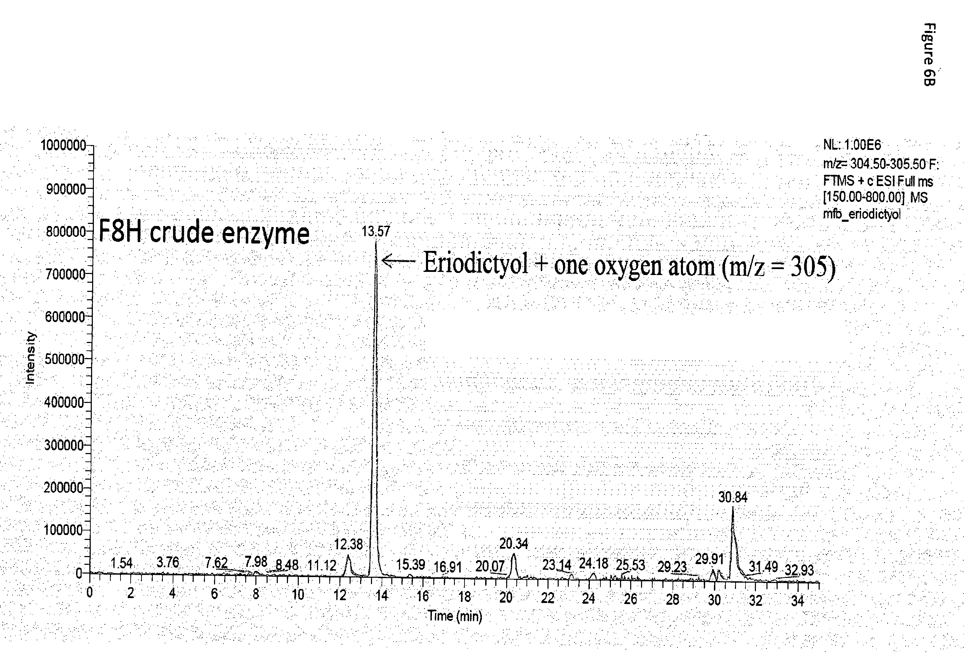

The protein of the present invention also possesses the activity to hydroxylate flavanones such as eriodictyol, etc., and flavones such as luteolin, etc. (FIG. 6 - FIG. 10).

In addition, the protein of the present invention is a flavin enzyme protein. In the present invention, the term "flavin enzyme" is intended to mean an oxidoreductase requiring a riboflavin such as FAD, FMN, etc. as a coenzyme.

Therefore, the protein of the present invention is "the flavin enzyme protein having the flavonol 8-hydroxylase activity" and intended to mean a protein showing the flavonol 8-hydroxylase activity in the presence of a riboflavin such as FAD or FMN.

The protein to be tested is reacted with substrate flavonol in the presence or absence of a riboflavin (e.g., FAD or FMN). When the protein to be tested shows a higher flavonol 8-hydroxylase activity in the presence of the riboflavin than in the absence f the riboflavin, it can be determined that the protein to be tested is "the flavin enzyme protein having the flavonol 8-hydroxylase activity." The method for detecting the flavonol 8-hydroxylase activity is as described hereinbefore.

The flavin enzyme of the present invention is expected to have the Rossmann fold structure (Rossmann fold, Rao S, Rossmann M (1973). J Mol Biol 76 (2): 241-56), which mediates the binding to FAD and NAD(P).

The deletion, substitution, insertion and/or addition of one or more amino acid residues in the amino acid sequence of the protein of the invention is intended to mean that one or a plurality of amino acid residues are deleted, substituted, inserted and/or added at one or a plurality of positions in the same amino acid sequence. Two or more types of the deletion, substitution, insertion and addition may occur concurrently.

Hereinbelow, examples of the amino acid residues which are mutually substitutable are given below. Amino acid residues in the same group are mutually substitutable. Group A: leucine, isoleucine, norleucine, valine, norvaline, alanine, 2-aminobutanoic acid, methionine, o-methylserine, t-butylglycine, t-butylalanine and cyclohexylalanine; Group B: aspartic acid, glutamic acid, isoaspartic acid, isoglutamic acid, 2-aminoadipic acid and 2-aminosuberic acid; Group C: asparagine and glutamine; Group D: lysine, arginine, ornithine, 2,4-diaminobutanoic acid and 2,3-diaminopropionic acid; Group E: proline, 3-hydroxyproline and 4-hydroxyproline; Group F: serine, threonine and homoserine; and Group G: phenylalanine and tyrosine.

The protein of the present invention may also be produced by chemical synthesis methods such as the Fmoc method (fluorenylmethyloxycarbonyl method), the tBoc method (t-butyloxycarbonyl method), etc. In addition, peptide synthesizers available from Advanced Automation Peptide Protein Technologies, Perkin Elmer, Protein Technology Instrument, PerSeptive, Applied Biosystems, SHIMADZU Corp., etc. may also be used for the chemical synthesis.

### 3. Vector of the Invention and Vector-Introduced Transformants

In another embodiment, the present invention provides the expression vector comprising the polynucleotide of the present invention.

The vector of the invention is generally constructed to contain:
(i) a promoter that is transcribable in a host cell;
(ii) any of the polynucleotides described in (a) to (e) above that is linked to the promoter; and,
(iii) an expression cassette comprising as a component a signal that functions in the host cell with respect to the transcription termination and polyadenylation of RNA molecule.

The vector thus constructed is introduced into a host cell. The method for constructing the expression vector includes a method using a plasmid, phage, cosmid, etc., but is not particularly limited thereto.

The vector is not particularly limited to specific types and may be appropriately chosen from vectors that can be expressed in host cells. In other words, a promoter sequence is appropriately chosen to ensure the expression of the polynucleotide of the present invention depending upon type of host cells; the promoter sequence and the polynucleotide of the present invention are incorporated into various plasmids, etc., and the resulting vectors may be used as expression vectors.

The expression vector of the present invention contains expression regulatory regions (e.g., a promoter, terminator and/or replication origin, etc.) depending upon type of the host to be introduced. As the promoter for bacteria, there are employed conventional promoters (e.g., a trc promoter, tac promoter, lac promoter, etc.). As the promoter for yeast, a glyceraldehyde 3-phosphate dehydrogenase promoter, PH05 promoter, etc. may be used. The promoter for filamentous fungi includes, for example, promoters of amylase, trpC, etc. Examples of the promoter to express the target gene in plant cells include 35S RNA promoter of cauliflower mosaic virus, rd29A gene promoter, rbcS promoter, mac-1 promoter produced by adding the enhancer sequence of the cauliflower mosaic virus 35S RNA promoter to the 5' end of the mannopine synthetase promoter sequence derived from Agrobacterium. The promoter for animal cell hosts includes viral promoters (e.g., SV40 early promoter, SV40 late promoter, etc.).

As a selection marker used for the transformation, there may be used auxotrophic markers (ura5, niaD), chemical-resistant markers (hygromycin, zeocin), genecitin-resistant gene (G418r), copper-resistant gene (CUP1) (Marin et al., Proc. Natl. Acad. Sci. USA, vol. 81, p. 337, 1984), cerulenin-resistant gene (fas2m, PDR4) (Junji Inokoshi, et al., Biochemistry, vol. 64, p. 660, 1992; Hussain et al., Gene, vol. 101, p. 149, 1991, respectively).

The present invention further provides the transformants, into which the polynucleotide of the present invention (e.g., the polynucleotide according to any one of (a) to (e) described above) is introduced.

The method of constructing (method of producing) the transformants is not particularly limited and includes, for example, a method which comprises introducing the aforesaid recombinant vector into a host followed by transformation. The host cells as used herein are not particularly limited and various cells hitherto known may be advantageously used. Specific examples include, but not limited to, bacteria such as Escherichia coli, etc., yeasts (Saccharomyces cerevisiae, Schizosaccharomyces pombe), plant cells, animal cells, etc. Culture media and conditions suitable for the host cells described above are well known in the art. The organism to be transformed is not particularly limited either and includes various microorganisms, plants and animals given as examples of the host cells above.

For transformation of the host cell, there may be used generally known methods. For example, methods which can be used include but not limited to the electroporation method (Mackenzie, D. A. et al., Appl. Environ. Microbiol., vol. 66, p. 4655-4661, 2000), the particle delivery method (method described in JPA 2005-287403 "Method of Breeding Lipid-Producing Fungus"), the spheroplast method (Proc. Natl. Acad. Sci. USA, vol. 75, p. 1929, 1978), the lithium acetate method (J. Bacteriology, vol. 153, p. 163, 1983), and methods described in Methods in yeast genetics, 2000 Edition: A Cold Spring Harbor Laboratory Course Manual, etc.

For other general molecular biological techniques, reference can be made to Sambrook & Russell, Molecular Cloning: A Laboratory Manual Vol. 3, Cold Spring Harbor Laboratory Press 2001," "Methods in Yeast Genetics, A laboratory manual (Cold Spring Harbor Laboratory Press, Cold Spring Harbor, NY)," etc.

In an embodiment of the present invention, the transformant may be a plant transformant. The plant transformant in accordance with the embodiment can be acquired by introducing a recombinant vector comprising the polynucleotide of the present invention into a plant in such a manner that the polypeptide encoded by the polynucleotide of the present invention can be expressed.

Where a recombinant expression vector is used, the recombinant expression vector used to transform the plant is not particularly limited as far as the vector is capable of expressing the polynucleotide of the present invention in said plant. Examples of such vectors include a vector bearing a promoter capable of constitutively expressing the polynucleotide in plant cells, and a vector bearing a promoter inducibly activated by external stimulation.

Examples of the promoter constitutively expressing the polynucleotide in plant cells include 35S RNA promoter of cauliflower mosaic virus, rd29A gene promoter, rbcS promoter, mac-1 promoter, etc.

Examples of the promoter inducibiy activated by external stimulation include mouse mammary tumor virus (MMTV) promoter, tetracycline-responsive promoter, metallothioinene promoter, heat shock protein promoter, etc.

Where the host lack the ability to synthesize flavonols, the polynucleotide of the present invention may be expressed in host and at the same time, a gene encoding flavonol synthase (FLS) may be expressed therein. In such a case that a plurality of exogenous genes are expressed in the same host, for example, the polynucleotide of the present invention is inserted into one expression vector and the flavonol synthase gene is inserted into another expression vector. Each of these plural expression vectors may be introduced into the host. Alternatively, the polynucleotide of the present invention and the flavonol synthase gene may be incorporated into the same expression vector, and the resulting expression vector may be introduced into the host. Where a plurality of inserts are expressed on one expression vector as in the latter case, each insert may also be expressed by the same promoter (using, e.g., the IRES (internal ribosome entry site) system), or may be independently expressed by different promoters. The vector capable of independently expressing plural inserts can be constructed using, e.g., Gateway (registered trademark) System (Invitrogen).

Plants that are subject to transformation in the present invention are intended to mean entire plant bodies, plant organs (e.g., leaves, petals, stems, roots, seeds, etc.), plant tissues (e.g., epidermis, phloean, parenchyma, xylem, vascular bundles, palisade tissues, spongy tissues, etc.) or plant culture cells, or may be any of various types of plant cells (e.g., suspension culture cells), protoplasts, leaf slices, calli, and the like. Plant species which are used for transformation are not particularly limited and may be any plant from those belonging to the Monocotyledoneae or the Dicotyledoneae.

Conventional transformation methods (e.g., the Agrobacterium method, gene gun method, PEG method, electroporation method, etc.) known to those ordinarily skilled in the art are used to transform genes to plants. For example, the Agrobacterium-mediated method and the method of directly introducing into plant cells are well known. When the Agrobacterium method is used, the plant expression vector constructed is introduced into an appropriate Agrobacterium strain (e.g., Agrobacterium tumefaciens), followed by infection of aseptically cultured leaf discs with this strain according to the leaf disc method (Hirobumi Uchimiya, Manuals for Plant Gene Manipulation (1990), pp. 27-31, Kodansha Scientific Co., Ltd., Tokyo), etc. Thus, transgenic plant can be acquired. The method by Nagel, et al. (Microbiol. Lett., 67: 325 (1990)) may also be used. This method involves introducing first, e.g., an expression vector into Agrobacterium and then introducing the transformed Agrobacterium into plant cells or plant tissues by the method described in Plant Molecular Biology Manual (Gelvin, S.B. et al., Academic Press Publishers). Herein, the "plant tissue" includes calli obtained by culturing plant cells. When the transformation is carried out using the Agrobacterium method, binary vectors (pBI121 or pPZP202, etc.) may be used.

For direct transfer of genes to plant cells or plant tissues, the electroporation method and the gene gun method are known. When a particle gun is used, plant bodies, plant organs or plant tissues per se may be used, or slices may be prepared and then provided for use, or protoplasts may be prepared and then provided for use. The samples thus prepared can be bombarded using a gene transfer apparatus (e.g., PDS-1000 (BIO-RAD, Inc.), etc.). Bombardment conditions may vary depending upon plants or samples. Normally, the bombardment is performed under a pressure of about 450 to 2000 psi at a distance of about 4 to 12 cm.

The cells or plant tissues into which the gene is introduced are first selected for their chemical resistance such as hygromycin resistance, etc. and then regenerated into plant bodies in a conventional manner. Regeneration of plant bodies from the transformant cells can be performed by methods known to those skilled in the art, depending upon species of plant cells.

Where a plant culture cell is used as a host, transformation is preformed by introducing the recombinant vector into culture cells by the gene gun method, the electroporation method, etc. Calli, shoots, hairy roots, etc. resulted from the transformation can be used directly in cell culture, tissue culture or organ culture. Furthermore, they can be regenerated into plant bodies by conventional plant tissue culture methods through administration of plant hormones (e.g., auxin, cytokinin, gibberellin, abscisic acid, ethylene, brassinolide, etc.) at appropriate concentrations.

Whether the gene is introduced into the host or not can be confirmed by PCR, Southern hybridization, northern hybridization, or the like. For example, a DNA is prepared from the transgenic plant and DNA-specific primers are designed to perform PCR. PCR can be performed under the same conditions as used for the preparation of plasmids described above. Subsequently, the amplified product is subjected to agarose gel electrophoresis, polyacrylamide gel eleetrophoresis, capillary electrophoresis, etc. and stained with ethidium bromide, SYBR Green solution, etc. By detecting the amplified product as a single band, it can be confirmed that transformation has occurred. Alternatively, PCR may be performed using primers previously labeled with a fluorescent dye or the like, and the amplified product can then be detected. In addition, there may be employed such a method that the amplified product is bound to the solid phase of a microplate or the like to confirm the amplified product by fluorescence or enzyme reactions, or the like.

Once the transgenic plant where the polynucleotide in accordance with the present invention has been incorporated into the genome is acquired, its progeny can be obtained by sexual or asexual reproduction of the plant body. Also, the plant body can be mass-produced by acquiring from the plant body or its progeny or clones thereof, e.g., seeds, fruits, cut panicles, tubers, tuberous roots, strains, calli, protoplasts, etc., and then using them as the origin. Accordingly, the present invention further includes the plant body in which the polynucleotide in accordance with the present invention is expressibly introduced, or progenies of the plant body having the same property as in the plant body, and tissues derived therefrom.

Moreover, the transformation methods for various plants are already reported. Examples of the transgenic plants in accordance with the present invention include, but not be limited to, solanaceous plants (e.g., eggplant, tomato, green pepper, potato, tobacco, datura or downy thorn apple, alkakengi, petunia, Calibrachoa sp., nierembergia, etc.), leguminous plants (e.g., soybean, azuki bean, peanut, common bean or Phaseolus vulgaris, broad bean, Lotus japonicus, etc.), rosaceous plants (e.g., strawberry, plum, cherry, rose, blueberry, blackberry, bilberry, cassis, raspberry, etc.), caryophyllaceous plants (carnation, soap root, etc.), chrysanthemum plants (chrysanthemum, gerbera, sunflower, daisy, etc.), orchidaceous plants (orchid, etc.), primulaceous plants (cyclamen, etc.), gentianaceous plants (lisianthus, gentian, etc.), iridaceous plants (freesia, iris, gladiolus, etc.), scrophulariaceous plants (antirrhinum, torenia, etc.), Kalanchoe pinnata (Kalanchoe), liliaceous plants (lily, tulip, etc.), convolvulaceous plants (morning glory, cairo morning glory, moonflower, sweet potator, Ipomoea quamoclit, Evolvulus or American blue, etc.), hydrangea plants (hydrangea, deutzia, etc.), cucurbitaceous plants (bottle gourd, etc.), geraniaceous plants (pelargonium, geranium, etc.), oleaceous plants (forsythia, etc.), vitaceous plants (e.g., grapevine, etc.), theaceous plants (camellia, tea, etc.), poaceous plants (e.g., rice plant, barley, wheat, oat, rye, sweet corn; foxtail millet, Japanese millet, kaoliang, sugar cane, bamboo, oat, finger millet, sorghum, Indian rice, Job's tears, pasture grass, etc.), moraceous plants (mulberry, hopvine, kouzo or paper mulberry, rubber tree, Cannabis, etc.), rubiaceous plants (Arabian coffee, gardenia, etc.), fagaceous plants (oak, Buna or Japanese beech, Kashiwa oak, etc.), Pedaliaceae plants (sesame, etc.), rutaceous plants (e.g., Daidai orange, Yuzu lemon, Unshu citrus, Japanese prickly ash) and brassicaceous plants (red cabbage, flowering cabbage, Japanese radish, Arabidopsis, rapeseed, cabbage, broccoli, cauliflower, etc.). Preferred examples of the plant include plants that are not reported so far to produce yellow coloured flowers, e.g., hydrangea, morning glory, petunia, geranium, begonia, cyclamen, saintpaulia, impatiens, and so on.

The plant transformed by the polynucleotide of the present invention (hereinafter "the plant of the present invention" or "the plant body of the present invention") abundantly contains the flavin enzyme protein having the flavonol 8-hydroxylate activity and, flavonols present in the plant body, especially in petals or calyxes are hydroxylated at position 8, resulting in conversion to flavonoid compounds that exhibit a yellow colour. Accordingly, the plant of the present invention can bloom flowers exhibiting a yellow colour.

Where the host plant used for transformation is a plant causing flowers to bloom only in colours other than yellow in the natural state, the plant is given to bloom new yellow coloured flowers not having existed heretofore. Therefore, the plant of the present invention is expected to provide a very high commercial value. Furthennore, flowers in different colour tones can be created by accumulating 8-hydroxylated flavonols in blue or red flowers.

The plant of the present invention can be appreciated or sold in any state of soil culture, pot culture, cut flowers or flowers only. Furthermore, only a part of flowers, e.g., corolla, petals or calyxes, can also be appreciated or sold. The plant of the present invention is expected to give a yellow colour not only in its flowers but also in their fruits. In the field of ornamental plants, not only flowers but also their fruits are appreciated for ornamental purposes. Accordingly, the fruits of the plant of the present invention are expected to have high commercial value.

Furthermore, the plant of the present invention can easily provide a complete plant by cultivating the seeds, cuttings, bulbs, etc. from the plant of the present invention.

Consequently, the plant of the present invention includes entire plant bodies, plant organs (e.g., leaves, petals, stems, roots, seeds, bulbs, etc..), plant tissues (e.g., epidermis, phloem, parenchyma, xylem, vascular bundles, palisade tissues, spongy tissues, etc.) or plant culture cells, or various types of plant cells (e.g., suspension culture cells), protoplasts, leaf slices, calli, and the like.

### 4. Processed Product of the Plant of the Invention

In these days, not only fresh flowers (e.g., soil-grown plants, pot plants, cut flowers, etc.) but also processed products of fresh flowers are sold as commercial products for plant appreciation. The plant of the present invention is very useful also as the material for such processed products of fresh flowers. Accordingly, in another embodiment of the present invention, the present invention provides processed products of the plant of the present invention (e.g., fresh flowers, cut flowers) or parts thereof (e.g., leaves, petals, stems, roots, seeds, bulbs, etc.). Examples of the processed products described above include, but not limited to, pressed flowers, dry flowers, preserved flowers, material flowers, resin-sealed products, etc.

### 5. Method for Producing Plant

By introducing the polynucleotide of the present invention into a plant body or parts thereof, the plant of the present invention can be produced. In an embodiment, therefore, the present invention provides the method for producing the plant of the present invention.

The method for producing the plant of the present invention may comprise the following steps.
(1) Step of introducing the polynucleotide of the present invention into a plant body or parts thereof:
   Introduction of the polynucleotide of the present invention into a host plant is the same as described above. The host plant may be any of entire plant bodies or, as parts thereof, plant organs (e.g., leaves, petals, stems, roots, seeds, etc.), plant tissues (e.g., epidermis, phloem, parenchyma, xylem, vascular bundles, palisade tissues, spongy tissues, etc.) or plant culture cells, or various types of plant cells (e.g., suspension culture cells), protoplasts, leaf slices, calli, and the like.
(2) Step of cultivation of the transgenic plant obtained in the step (1) above:
   Where the host plant used in the step (1) described above is a part of plant organs, plant tissues, plant cells, protoplasts, leaf slices or calli, the transformants may be cultivated under suitable environments to form perfect plant bodies. For the method for cultivation of a part of plant bodies to form perfect plant bodies, reference may be made to the description in the literature below: Seibutsu Kagaku Jikkenho (Laboratory Methods in Biological Chemistry) 41, Shokubutsu Saibo Kogaku Nyumon, published by Gakkai Shuppan Center, SBN 4-7622-1899-5.

### 6. Method for Screening Plant Which Blooms One or More Yellow Coloured Flowers

The present invention provides a method for screening plants which bloom one or more yellow coloured flowers. Specifically, the method comprises the following steps (1) to (3).
(1) extracting the polynucleotide from a subject plant;
(2) hybridizing the polynucleotide to a polynucleotide that hybridizes to a polynucleotide consisting of a nucleotide sequence complementary to the nucleotide sequence of the polynucleotide of the invention under stringent conditions; and,
(3) detecting the hybridization.

The step (1) described above can be performed by extracting the polynucleotide such as genomic DNA or mRNA, etc. from the subject plant. Parts of the subject plant, from which the polynucleotide such as genomic DNA, mRNA or the like is extracted, are not particularly limited and preferably, seeds. When mRNA is extracted, cDNA may be prepared from mRNA by reverse transcription.

The step (2) can be performed by hybridizing the polynucleotide extracted above under stringent conditions using the polynucleotide or oligonucleotide consisting of the nucleotide sequence complementary to the polynucleotide of the present invention as a probe or primer. The stringent conditions are the same as described above. The polynucleotide or oligonucleotide has a length of preferably 5 to 500 bp, more preferably, 10 to 200 bp, and most preferably, 10 to 100 bp. The polynucleotide or oligonucleotide can be easily synthesized by using various automated synthesizers (e.g., AKTA oligopilot plus 10/100 (GE Healthcare)), or by outsourcing to a third-party organization (e.g., Promega Corp. or Takara Bio Inc.).

Where the polynucleotide consisting of the nucleotide sequence complementary to the polynucleotide of the present invention is used as a probe in the step (2), the step (3) can be performed by ordinary methods for detecting hybridization, including Southern blotting, northern blotting (Sambrook, Fritsch and Maniatis, "Molecular Cloning: A Laboratory Manual" 2nd Edition (1989), Cold Spring Harbor Laboratory Press), Microarray (Affymetrix Inc.; cf., U. S. Patent Nos. 6,045,996 and 5,858,659), TaqMan PCR (Sambrook, Fritsch and Maniatis, "Molecular Cloning: A Laboratory Manual" 2nd Edition (1989), Cold Spring Harbor Laboratory Press), Fluorescent In Situ Hybridization (FISH) (Sieben V.J. et al., (2007-06). IET Nanobiotechnology 1 (3): 27-35), etc. On the other hand, where the polynucleotide consisting of the nucleotide sequence complementary to the polynucleotide of the present invention is used as a primer in the step (2), the hybridization can be detected in the step 3 by performing PCR amplification and analyzing the resulting amplified product through electrophoresis or sequencing (Sambrook, Fritsch and Maniatis, "Molecular Cloning: A Laboratory Manual" 2nd Edition (1989), Cold Spring Harbor Laboratory Press), etc.

### 7. Method for Producing Protein of the Invention

In a further embodiment, the present invention also provides the method for producing the protein of the present invention using the transformant described above.

Specifically, the protein of the present invention can be produced by isolating and purifying the protein of the invention from the culture of the transformant described above. As used herein, the term culture is intended to mean any of culture fluid, cultured bacterial cells or cultured cells, or disrupted cells of the cultured bacterial cells or cultured cells. The protein of the present invention can be isolated and purified in a conventional manner.

Specifically, where the protein of the present invention accumulates in cultured bacterial cells or cultured cells, the bacterial cells or cells after incubation are disrupted in a conventional manner (e.g., ultrasonication, lysozyme, freezing and thawing, etc.), and a crude extract of the protein of the invention can then be obtained in a conventional manner (e.g., centrifugation, filtration, etc.). Where the protein of the invention accumulates in culture fluid, the bacterial cells or cells can be isolated from the culture supernatant in a conventional manner (e.g., centrifugation, filtration, etc.) after completion of the culture to obtain the culture supernatant containing the protein of the invention.

The protein of the invention contained in the resulting extract or culture supernatant can be purified by means of conventional methods for isolation and purification. Examples of the methods for isolation and purification include ammonium sulfate precipitation, gel filtration chromatography, ion exchange chromatography, affinity chromatography, reversed phase high performance liquid chromatography, dialysis, ultrafiltration and the like; these methods can be appropriately used either alone or in combination thereof.

### 8. Method for Producing 8-Hydroxylated Flavonol

The protein of the present invention has the activity to hydroxylate flavonols at position 8. Thus, 8-hydroxylated flavonols can be efficiently produced by using the protein of the present invention.

Accordingly, the present invention provides the method for producing 8-hydroxylated flavonols, which comprises reacting the protein of the present invention or a fragment of the protein of the invention having the flavonol 8-hydroxylase activity with flavonols.

According to the present invention, mixing of the protein, etc. of the invention with flavonols may be sufficient for the reaction of the protein of the present invention or a fragment of the protein of the invention having the flavonol 8-hydroxylase activity (hereinafter "the protein, etc. of the invention") with flavonols. After mixing them, the mixture is preferably stirred gently (using a shaker, a rotor, etc.). Preferably, the method of the present invention is carried out at such a temperature that the flavonol 8-hydroxylase activity of the protein, etc. of the invention can be fully exhibited, e.g., at an ambient temperature (approximately 25°C).

The 8-hydroxylated flavonols are useful not only as pigments but also as raw materials for drugs having an antioxidant activity. For example, gossypin with sugar molecule attached to the hydroxyl group at position 8 of gossypetin is known to possess an anti-inflammatory activity and anti-tumor activity and reportedly inhibits the activation of nuclear factor-κB (NF-κB) leading to cell apoptosis potentiation (Non-Patent Document 2). It is also reported that gossypin upregulates low density lipoprotein receptor (LDLR) through activation of extracellular signal-regulated kinase (ERK) (Lu N. et al., (2008) J. Agric. Food Chem. 56 (23), 11526-11532). It is thus expected that gossypin improves disturbance of cholesterol metabolism.

### EXAMPLES

Hereinafter, the present invention is described by way of EXAMPLES but the scope of the invention is not deemed to be limited thereto.

### [EXAMPLE 1]

### Cloning of cDNA of flavonol 8-hydroxylase

cDNA was cloned by the following procedures.

Using the data from EST analysis by Kazusa DNA Research Institute (Asamizu et al., PMB, 54, 405-414, 2004), search was conducted on genes specifically expressed in the petals and buds upon flowering of Lotus japonicus. The resulting 2306 singletons and 321 clusters were screened and, clones strongly expressed in buds were selected therefrom.

Homology search using BlastX was carried out for 6 reading frames of DNA sequences of all these clones to select the clones of monooxygenase and an unknown protein. It was expected from Halbwirth and Stich, Phytochemistry 67 (2006) 1080-1087 (Non-Patent Document 5) that flavonol 8-hydroxylase (F8H) would have a FAD binding domain. The FAD binding domain was searched for the putative amino acid sequence for the candidate DNA sequence and it was found difficult to conduct search based on the amino acid sequence information using a software (Blast, FASTA, etc.) for comparing amino acid sequences. Accordingly, search was again conducted for the FAD binding domain, focusing attention on the secondary structure, not on the amino acid sequence. Specifically, it was performed by a search software or pfam search (http://pfam.sanger.ac.uk/search) to determine if the babab structure (beta-strand - alpha-helix - beta-strand - alpha-helix - beta-strand structure) which is characteristic of the Rossmann fold (Rao S., Rossmann M. (1973). J. Mol. Biol. 76 (2): 241-56) is present in the reading frame above. The DNA cluster KN1C019440A determined to have the FAD binding site was identified by this search. Surprisingly, the cluster was not observed in the gene expressed in the petals of blooming flowers. Also, the cluster was found to consist of five ESTs. Based on the result, the clone MFB088d08 having the longest translation sequence was obtained. This clone was cultured from Escherichia coli glycerol stock to obtain the plasmid. The full-length sequence was then determined. The sequence is shown by SEQ ID NO: 1. The cDNA sequence encoded by the clone is named MFB088.

Based on the nucleotide sequence of cDNA for F8H, genome gene sequence was searched. Specifically, clones containing gene sequences sharing the sequence identity with the nucleotide sequence of cDNA for F8H were searched from the BAC clone library of Lotus japonicus genome by Kazusa DNA Institute (Sato S. et al., (2008) DNA Research, pp. 1-13). The results revealed that the F8H gene was contained in the BAC clone LjB13E21 (BM2348) (88, 723 bp) and present on chromosome 3 in the Lotus japonicus genome (82.8 cM).

The positions of exons and introns of the F8H gene in the BAC clone LjB13E21 are shown in the table below, wherein the positions are indicated by the number of nucleotide residues by counting in order toward the 3` end when the nucleotide residue at the 5' end of Lotus japonicus genome is numbered as the first residue.

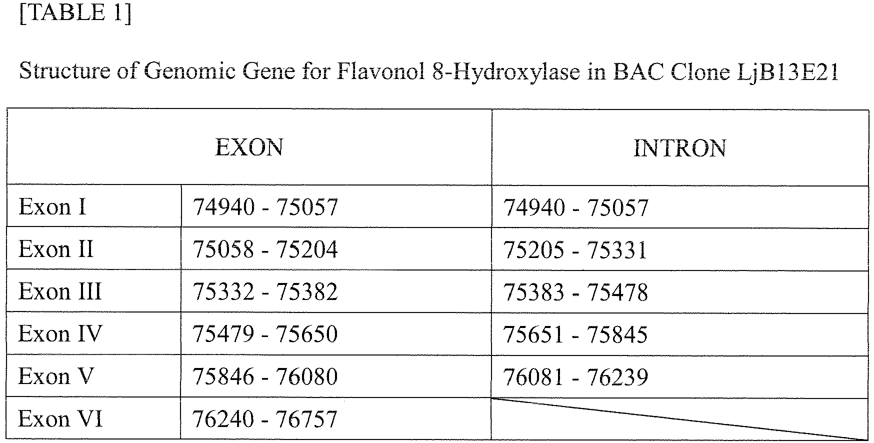

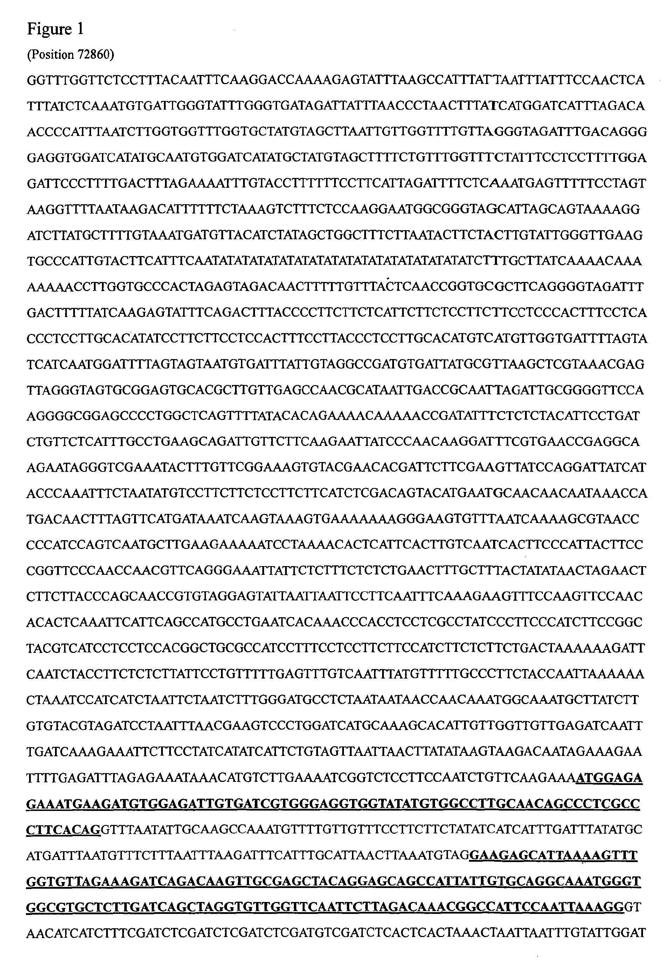

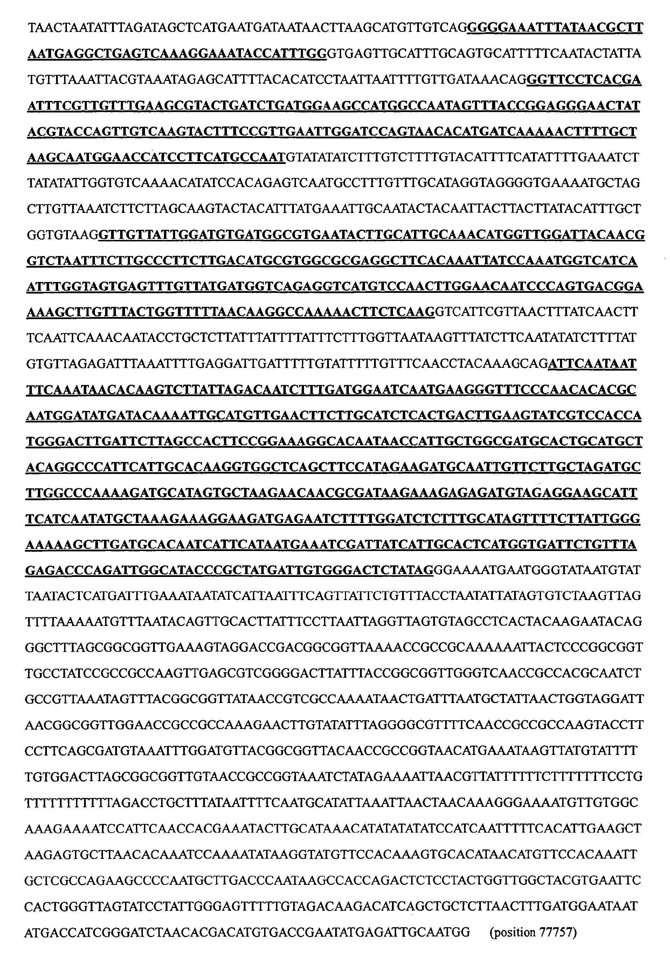

FIG. 1 shows the domain containing 2,000 bp upstream of the start codon and 1,000 bp downstream of the termination codon of the F8H gene in the nucleotide sequence of the BAC clone LjB13E21 of Lotus japonicus containing the F8H gene. In FIG. 1, the sequence regions shown by bold and underline denote the exon regions of the F8H gene. The nucleotide sequence from the start codon of exon I to the stop codon of exon VI in the Lotus japonicus F8H gene is shown in SEQ ID NO: 3.

Measurement of flavonol 8-hydroxylase activity

### (1) Preparation of transformed yeast

The cDNA cloned above was introduced into yeast cells to prepare transformed yeast.

To amplify the full-length translation region of the cloned cDNA, the following primers were prepared based on the sequence of the cDNA.
F8H-F primer: 5'-AAGAAAATGGAGAGAAATGAAGATGTGG-3' (SEQ ID NO: 4)
F8H-R primer: 5'-CTATAGAGTCCCACAATCATAGCGGG-3' (SEQ ID NO: 5)

Using the primers above and KOD-Plus- (TOYOBO Co. Ltd., Japan) as well as the cloned cDNA as a template, amplification was performed by PCR under the following conditions.
PCR conditions: [98°C: 15 secs., 60°C: 15 secs., 74°C: 30 secs.] x 15 cycles

Using Target Clone (TOYOBO), one adenine nucleotide was attached to the 5' ends of the cDNA fragment amplified by PCR. The cDNA after adenine addition was ligated to the TA-cloning site downstream of the galactose-inducible promoter GAL1 in the pYES2 TOPO vector (Invitrogen Corporation, California, USA) as a yeast expression vector . The ligation direction and sequence of the resulting plasmid pYESMFB088 were confirmed by the Sanger method.

The yeast strain BJ2168 (a; prc1-407, prbl-1122, pep4-3, leu2, trp1, ura3-52, Nippon Gene Co., Ltd.) was used as a host for protein expression. Using Frozen-EZ Yeast Transformation II Kit (Zymo Research, California, USA), the plasmid was introduced into the yeast, followed by transformation of the yeast according to the manual attached to the kit. The transformed yeast was plated on selection medium containing Yeast Nitrogen Base without Amino Acids (6.7 g/l, Invitrogen Corporation), glucose (20 g/l), leucine (30 mg/l), tryptophan (20 mg/l) and agar (20 g/l) (hereinafter, selection agar medium), followed by stationary culture at 30°C for 3 days. The yeast thus grown was used as transformants. Two types of transformed yeast below were prepared: (1) control yeast (pYES2 was introduced) and (2) F8H expression yeast (pYESMFB088 was introduced).

### (2) Preparation of MFB088 crude enzyme solution

The transformed yeast prepared above was cultured under the conditions below.

The transformed yeast grown on selection agar medium was aseptically inoculated on 20 ml of selection liquid medium (agar-free selection agar medium) and shake cultured for 24 hours under the conditions at 30°C and 200 rpm. After the shake culture, the transformed yeast was collected by centrifugation at 3000 x g for 10 minutes. Liquid medium containing Yeast Nitrogen Base without Amino Acids (6.7 g/l, Invitrogen), galactose (20 g/l), leucine (30 mg/l) and tryptophan (20 mg/l) (hereinafter, induction liquid medium) was used to induce protein expression. The total amount of the transformed yeast collected was suspended in 100 ml of induction liquid medium and shake cultured for 48 hours under the conditions at 30°C and 200 rpm to induce protein expression.

The yeast cells were centrifuged and recovered, and then lysed using glass beads. The lysate was centrifuged at 8000 x g for 10 minutes. The supernatant was recovered and further centrifuged at 15000 x g for 10 minutes to give the crude enzyme fraction of the protein encoded by MFB088 (hereinafter referred to as the MFB088 crude enzyme).

### (3) Preparation of sample for assaying the flavonol 8-hydroxylase activity

The reaction solution containing the following was prepared. MFB088 crude enzyme solution, 2 µl (containing 2.5 µg of proteins);
0.1 M KH₂PO₄/K₂HPO₄ solution (containing 0.4% ascorbic acid: pH 7.0), 88 µl;
0.048 nmol eriodictyol/5 µl of H₂O;
0.001 nmol FAD and 0.48 nmol NADPH/5 µl of H₂O.

NADPH was added to start the reaction. After the reaction proceeded at 30°C for 15 minutes, 10 µl of glacial acetic acid was added to stop the reaction. An acetic acid aqueous solution was further added to extract the phenol compound twice (the added acetic acid aqueous solution: 70 µl and 50 µl). The organic layer was recovered and used as a sample (hereinafter referred to as "'MFB088 crude enzyme reaction product"). The components were analyzed by UPLC-TOF MS and ion trap MS/MS.

In the same manner as described above, the organic phase was recovered from the yeast cells containing no insert, in which the pYES2 vector only was introduced; this was used as negative control for component analysis.

### (4) Ultra-performance liquid chromatography with time-of-flight mass spectrometry (UPLC/TOF MS)

The components of the MFB088 crude enzyme reaction product were analyzed by UPLC-TOF MS.

For analysis of the MFB088 crude enzyme reaction product, Waters Acquity UPLCTM system (Waters Corporation) equipped with time-of-flight mass spectrometer LCT Premier (Waters Corporation, Massachusetts, USA) was used. The analysis was performed using an ACQUITY UPLCTM BEH C18 column (particle diameter of 1.7 µm, column size of 2.1 mm I.D. x 100 mm, Waters Corporation) under the conditions of flow rate of 0.3 ml/min and column temperature at 40°C. Using solvent A (0.1 % formic acid-acetonitrile) and solvent B (0.1% formic acid aqueous solution), the reaction product was eluted by linear gradient from 70% B in 0 minute to 70% A in 20 minutes. The reaction product was detected by the time-of flight mass spectrometer in the cation detection mode set forth below: capillary voltage of 2500 V, sample cone of 100 V, desolvation temperature at 250°C, source temperature at 120°C, and nitrogen gas flow of 600 1/hour.

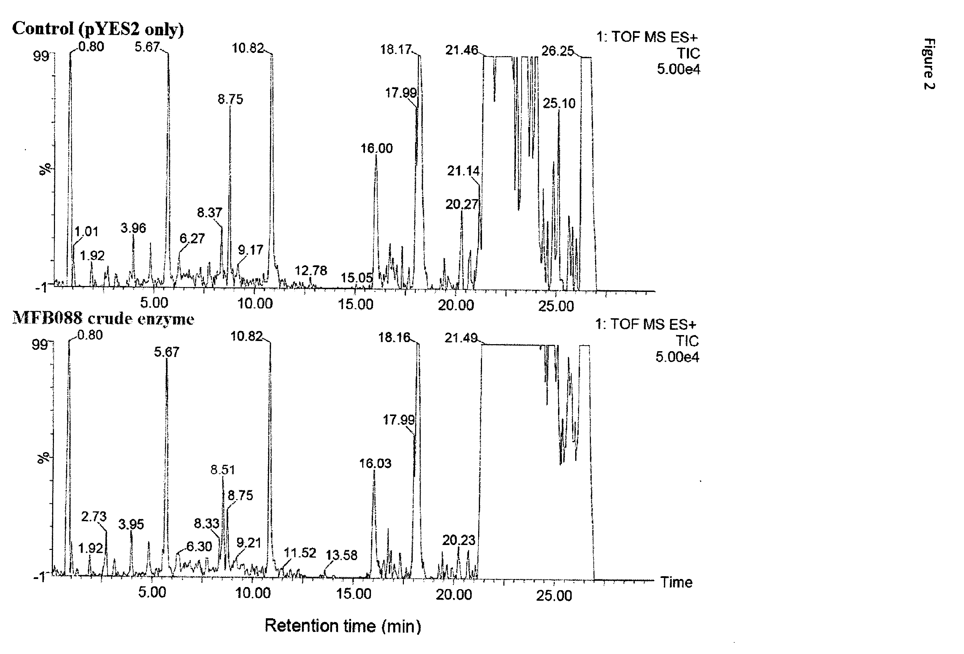

As a result, the peak corresponding to gossypetin (retention time: 8.51 min) was detected from the MFB088 crude enzyme reaction product. In contrast, any peak corresponding to gossypetin was not detected from negative control (FIG. 2).

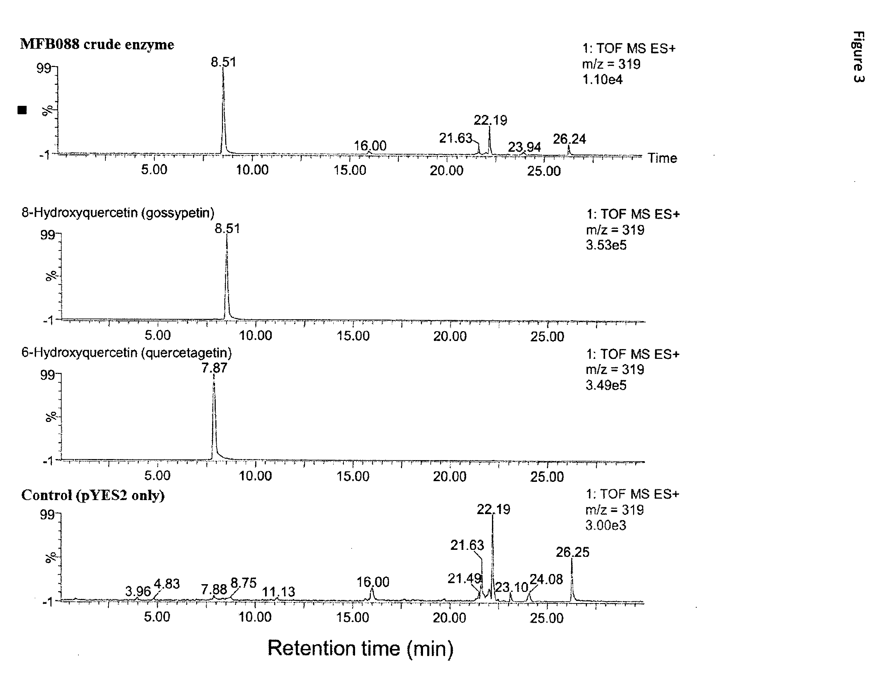

Next, comparison was made among the peaks for the MFB088 crude enzymes reaction product, 8-hydroxyquercetin (=gossypetin: standard) wherein position 8 of quercetin is substituted with hydroxyl group, 6-hydroxyquercetin (= quercetagetin) wherein position 6 of quercetin is substituted with hydroxyl group and negative control (FIG. 3).

In the MFB088 crude enzyme reaction product and 8-hydroxyquercetin, the peak was observed at retention time of 8.51 (min) but no peak was observed in 6-hydroxyquercetin and negative control (FIG. 3).

The components of the MFB088 crude enzyme reaction product was analyzed by ion trap MS/MS. High performance liquid chromatography (HPLC) system Agilent 1200 series (Agilent Technologies, California, USA) equipped with ion trap mass spectrometer LTQ XL (Thermo Fisher Scientific, Massachusetts, USA) was used for the MS/MS analysis. HPLC was performed using a TSKgel ODS-80TM column (particle diameter of 5 µm, column size of 4.6 mm I.D. x 150 mm, TOSOH, Japan) under the conditions of flow rate of 0.5 ml/min and column temperature at 40°C. Using solvent A (0.1% formic acid-acetonitrile) and solvent B (0.1% formic acid aqueous solution), the reaction product was eluted by linear gradient from 70% B in 0 minute to 85% A in 30 minutes. The reaction product was ionized by electrospray ionization method and cations were detected under the following conditions: spray voltage of 4.0 kV, capillary temperature at 300°C, nitrogen sheath of 40 units, and auxiliary gas of 15 units. In the MS/MS analysis of the reaction product [M+H]+, collision-induced dissociation (CID) was performed under the conditions below to detect the fragment peak: normalized collision energy of 35, activation Q of 0.25 and activation time for 30 ms.

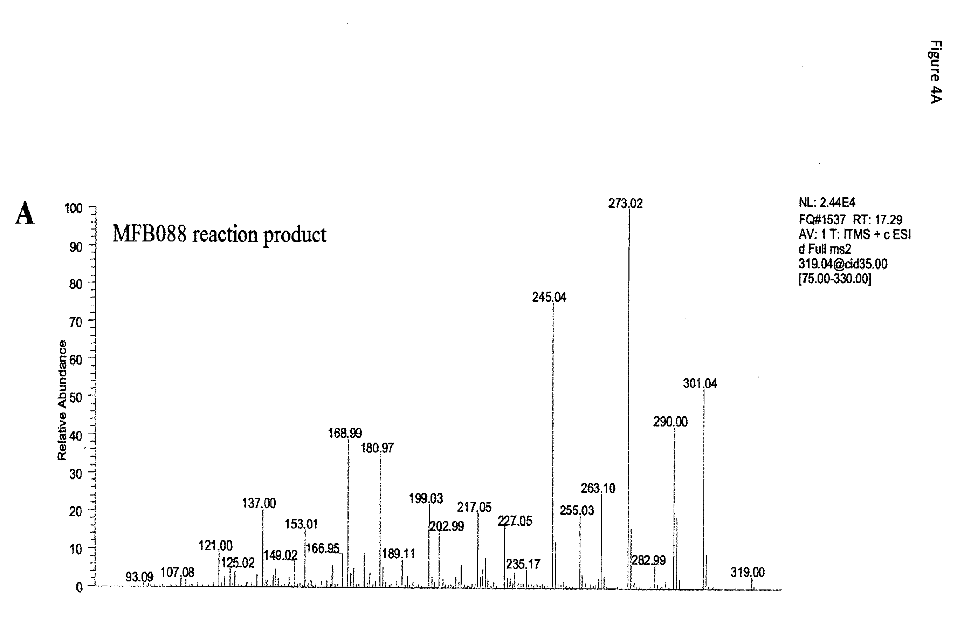

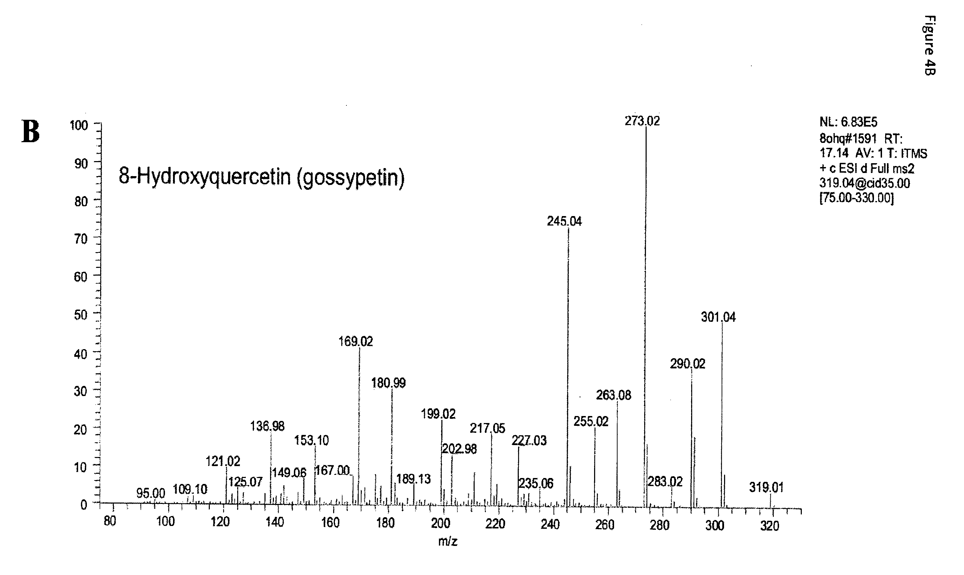

The peak patterns obtained were very similar between the MFB088 crude enzyme reaction product (FIG. 4A) and standard gossypetin (FIG. 4B).

The results of UPLC-TOF MS and ion trap MS/MS indicate that by the MFB088 crude enzyme, quercetin is substituted with hydroxyl group at position 8 and converted into gossypetin.

The MFB088 reaction product was measured for absorption wavelength MFB088.

Using a photodiode array of the HPLC system Agilent 1200 series (Agilent Technologies) used for the ion trap MS/MS described above, UV-visible light absorption of the eluates from 200 nm to 600 nm was detected. The column, solvents and elution conditions used for the analysis are the same as the conditions for the ion trap MS/MS described above.

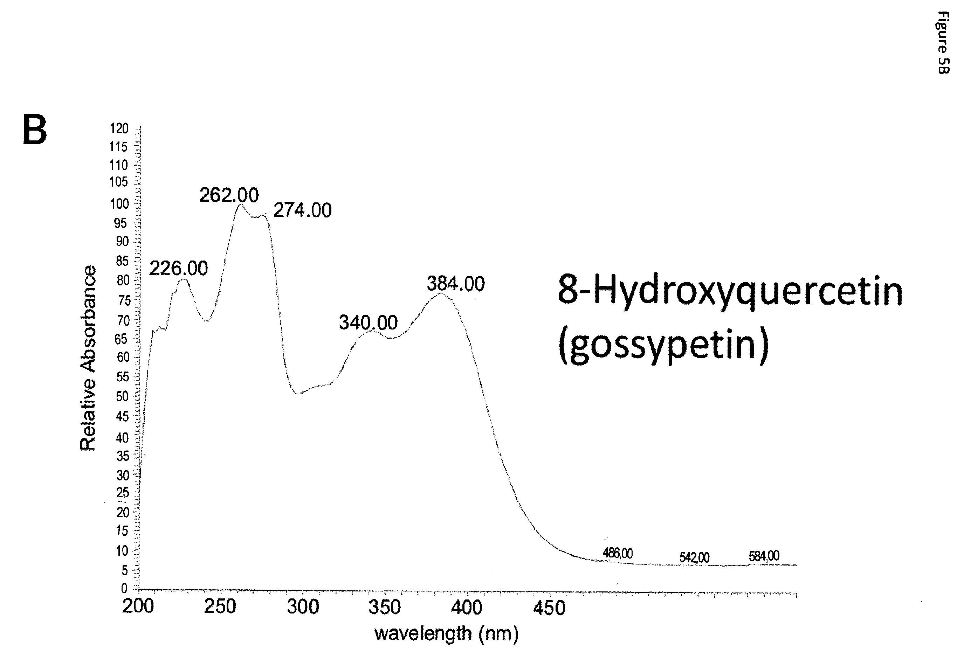

As shown in FIGS. 5A and 5B, the peak at 372.00 nm in the absorption peaks of quercetin is shifted to the absorption peak at 384.00 nm in gossypetin.

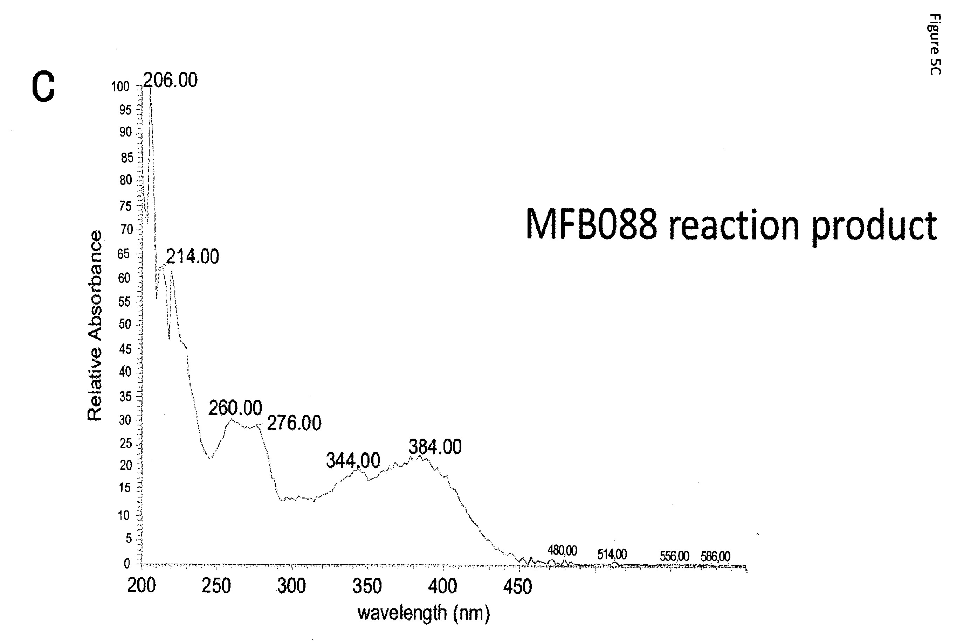

The MFB088 reaction product was measured for absorption wavelength and found to have the absorption peak at 384.00 nm as in gossypetin (FIG. 5C).

### [EXAMPLE 2]

### (1) Preparation of sample for assaying the flavanone hydroxylase activity

The reaction solution containing the following was prepared. MFB088 crude enzyme solution, 2 µl (containing 2.5 µg of proteins);
0.1 M KH₂PO₄/K₂HPO₄ solution (containing 0.4% ascorbic acid: pH 7.0), 88 µl;
0.048 nmoleriodictyol/5 µl of H₂O;
0.001 nmol FAD and 0.48 nmol NADPH/5 µl of H₂O.

After NADPH was added to start the reaction, the reaction product was recovered as in EXAMPLE 1. The components were analyzed by UPLC-TOF MS and ion trap MS/MS.

In the same manner as described above, the organic phase was recovered from the yeast cells containing no insert, into which the pYES2 vector only was introduced; this was used as negative control for component analysis.

### (2) Ultra-performance liquid chromatography with time-of-flight mass spectrometry (UPLC/TOF MS)

The components of the MFB088 crude enzyme reaction product were analyzed by UPLC-TOF MS as in EXAMPLE 1.

As a result, the peak corresponding to the molecule in which only one oxygen atom was attached to eriodictyol was detected from the MFB088 crude enzyme reaction product (retention time: 13.57 min). In contrast, any peak corresponding to the molecule described above was not detected from negative control (FIG. 6).

The mixture of the MFB088 crude enzyme solution and eriodictyol was analyzed by ion trap MS/MS as in EXAMPLE 1.

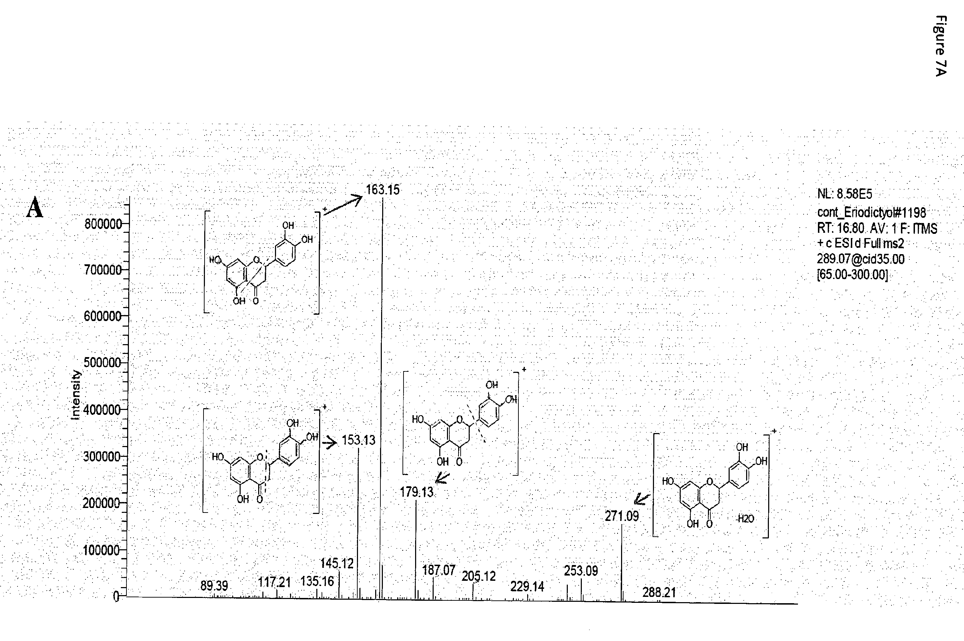

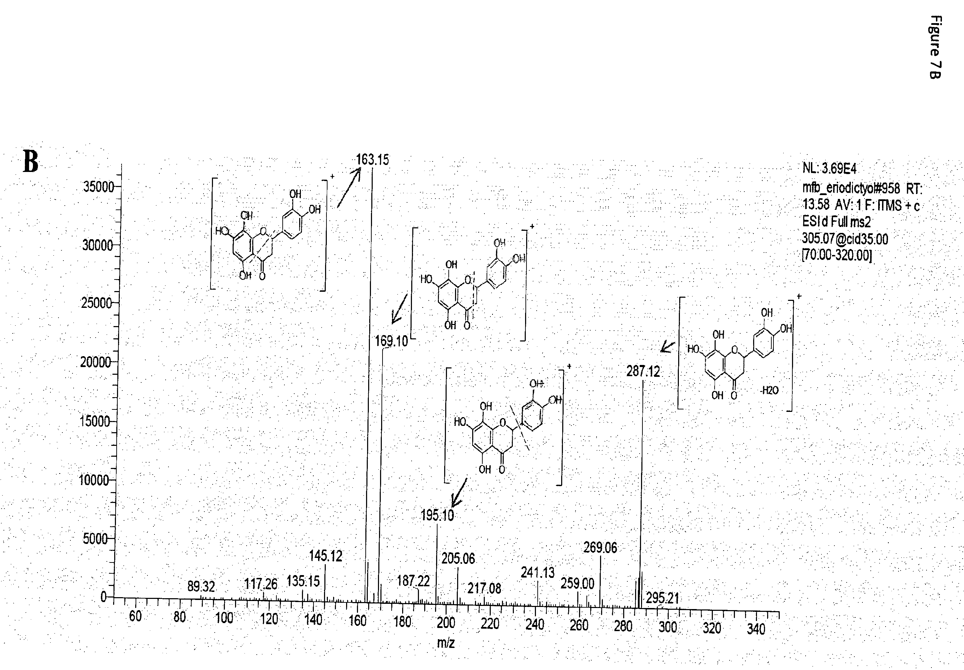

Next, the peaks of the mixture of the MFB088 crude enzyme solution and eriodictyol before and after the reaction were compared (FIG. 7A: before the reaction, FIG. 7B: after the redaction). It was found that the peak of the corresponding molecular structure was shifted by the addition of hydroxyl group. For example, the peaks of the molecular structure showing at 153.13 (m/z), 179.13 (m/z) and 271.09 (m/z) before the reaction were shifted to the peaks at 169.10 (m/z), 195.10 (m/z) and 287.12 (m/z), respectively, by the addition of hydroxyl group. It was found that the enzyme has the activity to hydroxylate eriodictyol, which is a flavanone.

The results of UPLC-TOF MS and ion trap MS/MS show that eriodictyol is hydroxyl-substituted by the MFB088 crude enzyme.

Next, the absorption wavelength of the MFB088 crude enzyme reaction product was measured as in EXAMPLE 1.

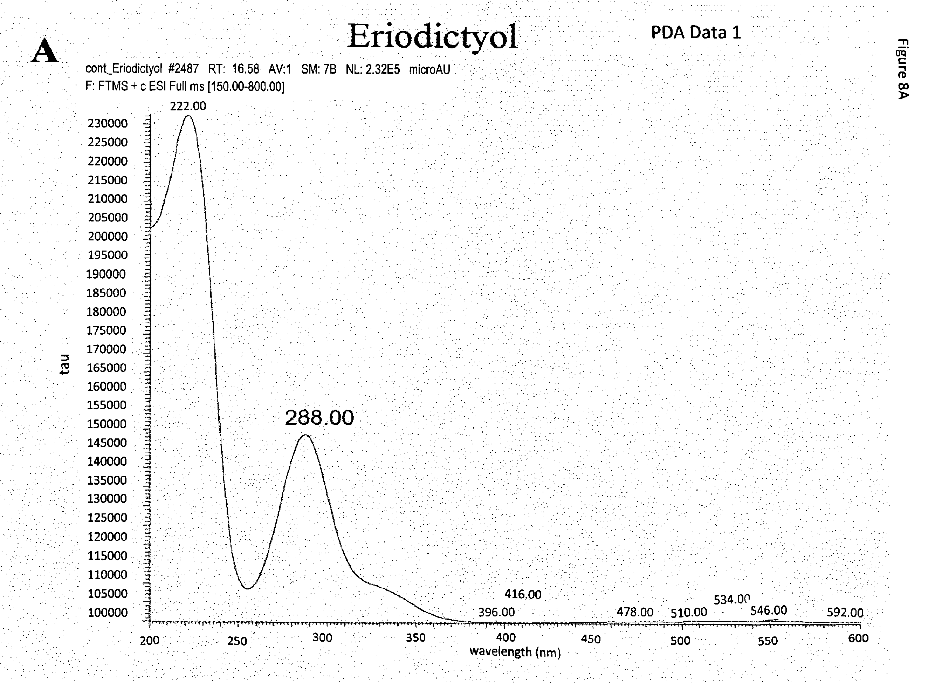

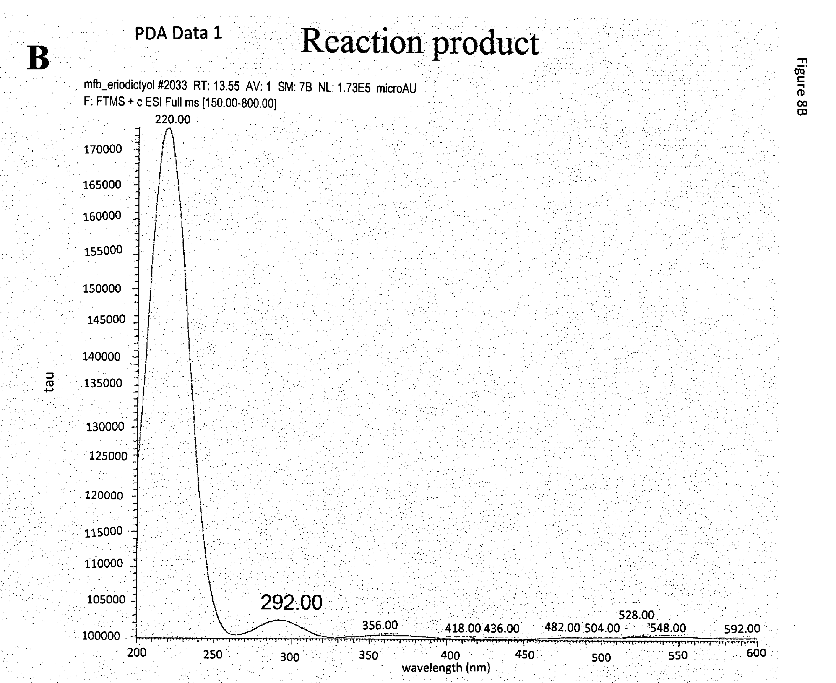

As shown in FIGS. 8A and 8B, the peak at 288.00 nm in the peaks eriodictyol has was shifted to the absorption peak at 292.00 nm after the reaction.

### [EXAMPLE 3]

### (1) Preparation of sample for assaying the flavone hydroxylase activity

The reaction solution containing the following was prepared.
MFB088 crude enzyme solution, 2 µl (containing 2.5 µg of protein);
0.1 M KH₂PO₄/K₂HPO₄ solution (containing 0.4% ascorbic acid: pH 7.0), 88 µl;
0.048 nmol luteolin/5 µl of H₂O;
0.001 nmol FAD and 0.48 nmol NADPH/5 µl of H₂O.

After NADPH was added to start the reaction, the reaction product was recovered as in EXAMPLE 1. The components were analyzed by UPLC-TOF MS and ion trap MS/MS.

In the same manner as described above, the organic phase was recovered from the yeast cells containing no insert, in which the pYES2 vector only was introduced; this was used as negative control for component analysis.

### (2) Ultra-performance liquid chromatography with time-of-flight mass spectrometry (UPLC/TOF MS)

The components of the MFB088 crude enzyme reaction product were analyzed by UPLC-TOF MS.

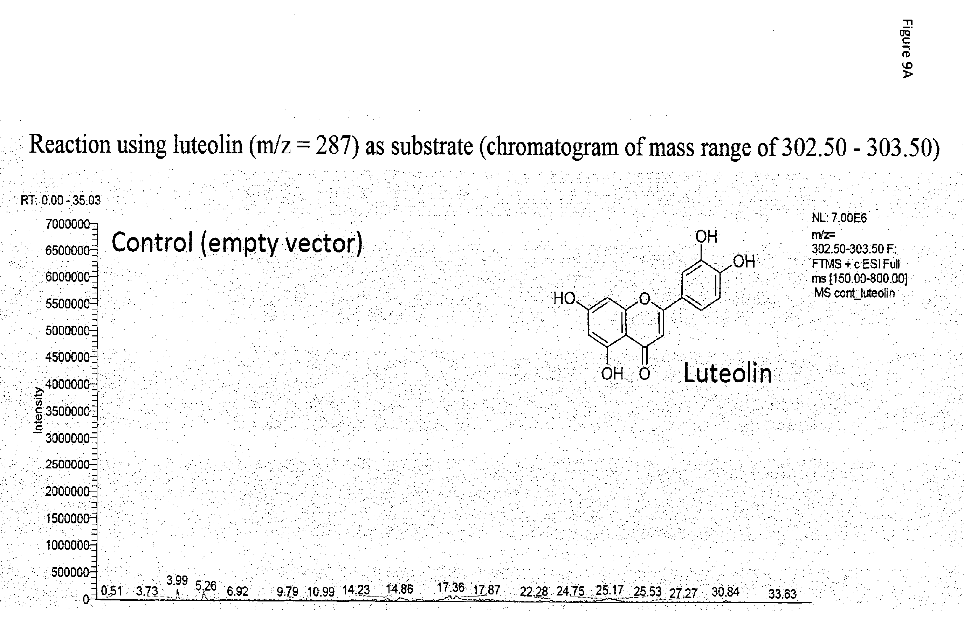

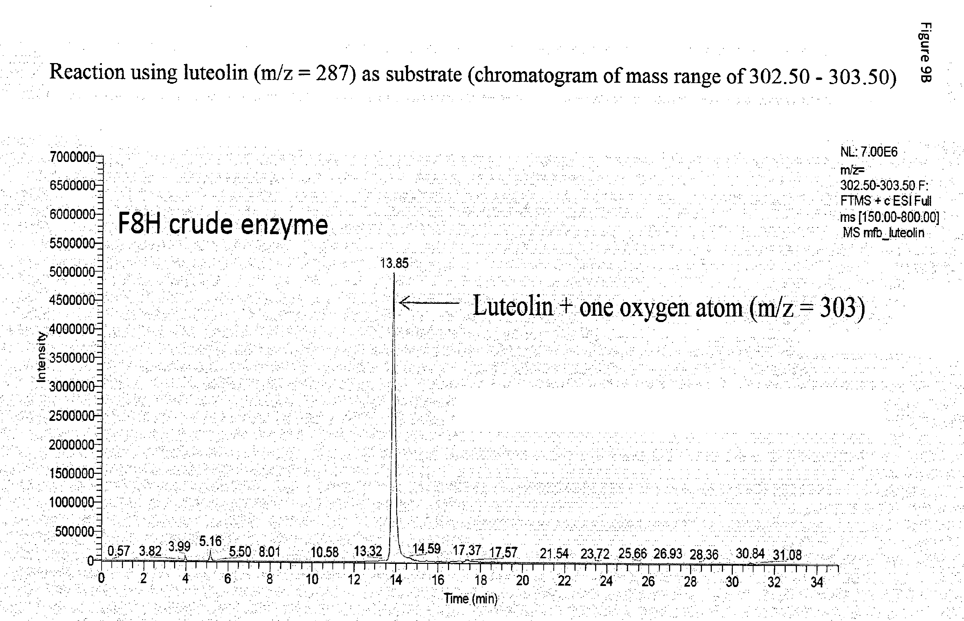

As a result, the peak corresponding to the molecule in which only one oxygen atom was attached to luteolin was detected from the MFB088 crude enzyme reaction product (retention time: 13.85 min). In contrast, any peak corresponding to the molecule described above was not detected from negative control (FIG. 9).

The mixture of the MFB088 crude enzyme solution and luteolin was analyzed by ion trap MS/MS as in EXAMPLE 1.

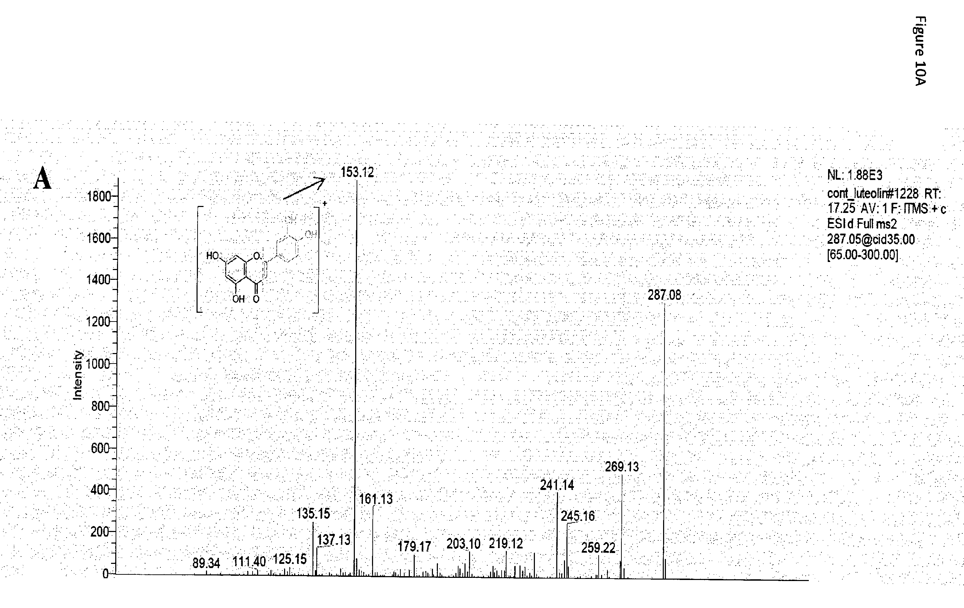

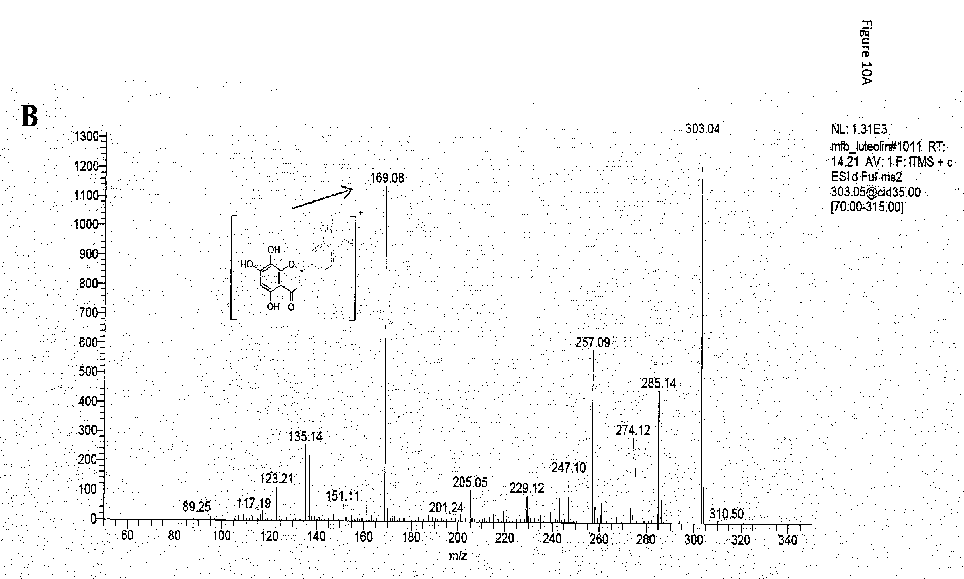

Next, the peaks of the mixture of the MFB088 crude enzyme solution and luteolin before and after the reaction were compared (FIG. 10A:11 before the reaction, FIG. 10B: after the reaction). It was found that the peak of the corresponding molecular structure was shifted by the addition of hydroxyl group. For example, the peak of the molecular structure showing at 153.12 (m/z) before the reaction was shifted to the peak at 169.08 (m/z) by the addition of hydroxyl group.

The results of UPLC-TOF MS and ion trap MS/MS reveal that hydroxyl group is attached to luteolin by the MFB088 crude enzyme.

### [EXAMPLE 4]

The reactivity requiring kaempferol and apigenin as substrates was analyzed. The reaction product was recovered in a manner similar to EXAMPLES described above and the components were analyzed by UPLC-TOF MS. As a result, the compound wherein hydroxyl group is attached to kaempferol at position 8 and the compound wherein hydroxyl group is attached to apigenin were observed.

When similar experiments were carried out using naringenin and dihydroquercetin as substrates, the compounds wherein two hydroxyl groups were attached to the respective substrates were detected.

Based on the foregoing results of EXAMPLES 1 to 4, it was confirmed that the polynucleotide of the present invention encodes the flavin enzyme protein having the flavonol 8-hydroxylase activity. It was also confirmed that the protein encoded by the polynucleotide of the present invention has the activity to hydroxylate position 8 of flavonols including quercetin and kaempferol. It was further confirmed that the protein encoded by the polynucleotide of the present invention has the activity to hydroxylate flavanones including eriodictyol and naringenin, flavones including luteolin and apigenin, dihydroflavonols including dihydroquercetin. Taking the regiospecificity of enzymes into account, these flavanones, flavones and dihydroflavonols are considered to be hydroxylated at position 8 thereof, as in the case where flavonols are used as substrate.

Furthermore, it was confirmed that the protein encoded by the polynucleotide of the present invention adds hydroxyl groups at two positions, when naringenin and dihydroquercetin are used as substrate. This indicates that the protein has the activity to hydroxylate at a different position, in addition to hydroxylation at position 8 of naringenin or dihydroquercetin.

### [EXAMPLE 5]

### Construction of the vector expressed in plant and preparation of transgenic plant

To construct the vector, restriction enzymes and Gateway System (Invitrogen Corp.) were used. The Gateway System was used according to the protocol provided by Invitrogen Corp.

The plasmid, which had a multicloning site between the E1235S promoter (described in WO 2005/017147) with the attL1 sequence (Invitrogen Corp.) added at the 5' end and mannopine synthetase terminator with the attR5 sequence (Invitrogen Corp.) added at the 3' end and had pDONR207 (Invitrogen Corp.) as the backbone, was named pSPB3868. The plasmid, in which F8H cDNA (MFB088: SEQ ID NO: 1) obtained in EXAMPLE 1 was inserted in forward orientation into the multicloning site, was named pSPB5205.

The plasmid, which had a multicloning site between the E1235S promoter and mannopine synthetase terminator and had pUCAP (described in WO 2005/017147) as the backbone, was named pSPB3647. The plasmid obtained by inserting the DNA fragment bearing flavonol synthase (FLS)cDNA (SEQ ID NO: 6) from rose in forward orientation into the multicloning site of pSPB3647, was named pSPB5207.

The sequence of the rose-derived flavonol synthase gene is available under DNA database: GenBank Accession No. AB038247. Acquisition of the rose-derived flavonol synthase gene is described in Tanaka Y, Fukuchi-Mizutani M, Mason JG (2003) Cloned genes. In: Roberts AV (ed) Rose Encyclopedia. Elsevier, Amsterdam, pp 341-347.

The plasmid, which had a multicloning site between the E1235S promoter with the attL5 sequence added at the 5' end and mannopine synthetase terminator with the attR2 sequence added at the 3' end and had pDONR207 as the backbone, was named pSPB3869. The DNA fragment bearing the E1235S promoter obtained by digesting pSPB5207 with AscI and PacI, rose-derived flavonol synthase cDNA and mannopin synthetase terminator was ligated to pSPB3869 digested with AscI and PacI to give pSPB5208.

Gateway cassette A (containing attR1 sequence and attR2 sequence, Invitrogen Corp.) was inserted into the Smal site of pUC19 to give pSPB3840. The DNA fragment containing Gateway cassette A obtained by digesting this plasmid with HindIII and SacI was inserted into pBinPLUS (described in WO 2005/017147) digested with HindIII and SacI, which was named pSPB3862.

A Gateway LR reaction was performed with pSPB5205, pSPB5208 and pSPB3862 to give the plasmid pSPB5213 for constitutively expressing F8H cDNA and the rose FLScDNA in plant cells. By infecting the leaf discs of Petunia hybrida line Skr4xSw63 (described in WO 93/01290) with Agrobacterium-transfected pSPB5213, transgenic petunia was generated. The method for transformation of petunia is disclosed in WO 93/01290.

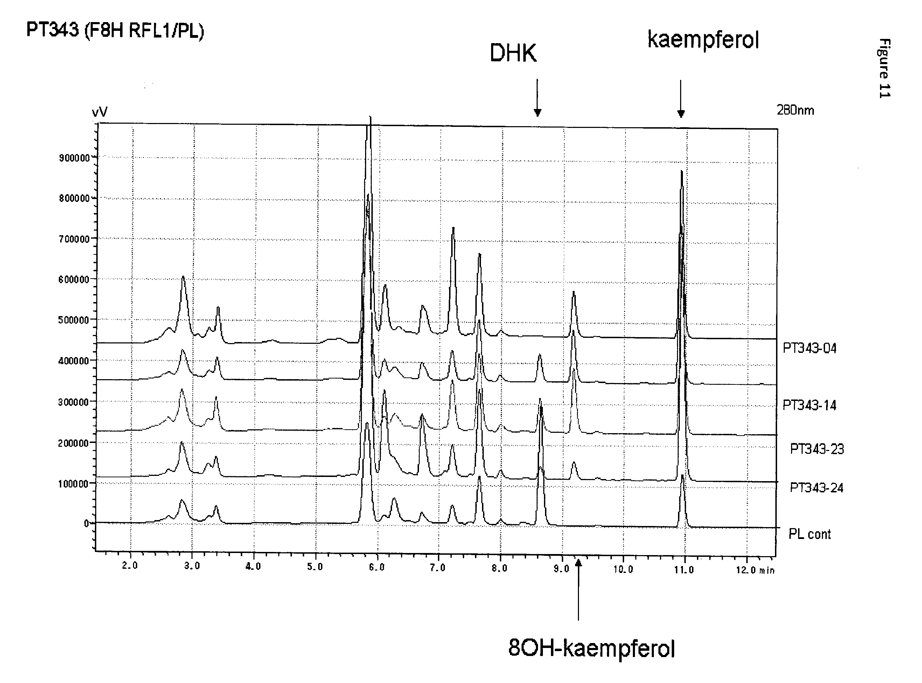

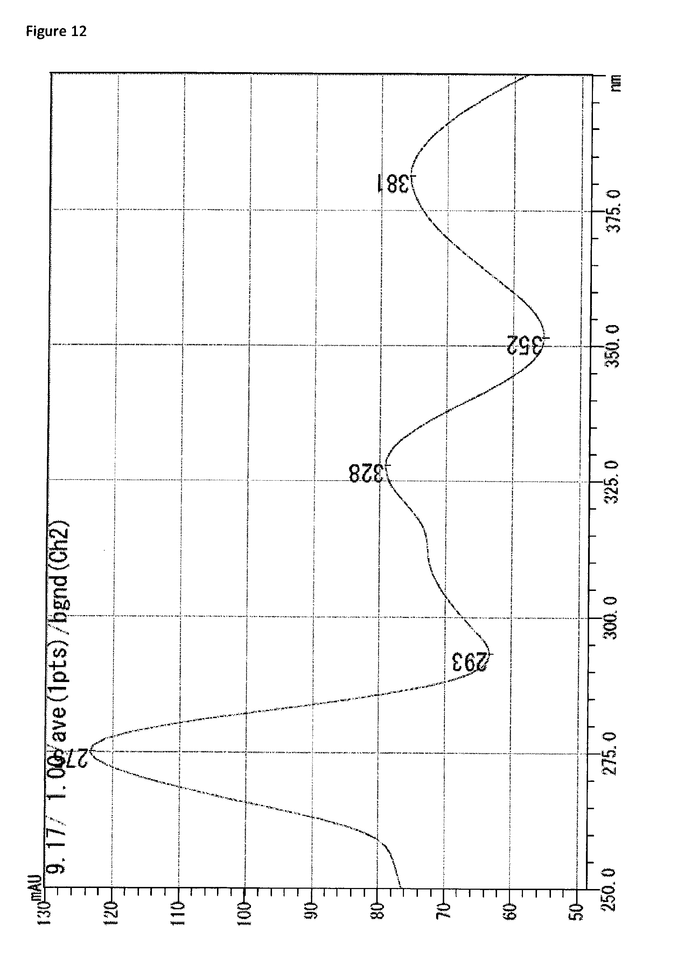

The flavonol obtained from the petunia petals were analyzed by high performance liquid chromatography according to the procedures disclosed in WO2008/156211. Under the conditions, kaempferol was eluted at approximately 10.8 minutes. The peak eluted at approximately 9.2 minutes, which was not found in the host petals, was observed in the genetically modified petunia (cf., FIG. 11). The absorption spectrum of this peak was measured and the absorption maximum was observed at 328 nm and 381 nm (cf., FIG. 12). The absorption maximum values of kaempferol were X and Y; the absorption maximum at 381 nm observed as a new peak was considered to be due to hydroxylation of kaempferol at position 8 as in the 8-hydroxylated quercetin described above.

A longer wavelength shift of this absorption maximum indicates that kaempferol changed to a compound exhibiting a stronger yellow colour (perceived by human eyes), by which it was demonstrated that the F8H gene obtained maintains and functions the activity also in the transgenic plant to form the 8-hydroxylated flavonol.

### Industrial Applicability

By introducing the polynucleotide of the present invention into plants, flavonol compounds in plants, especially petals, calyxes and fruits are hydroxylated at position 8 and change to flavonols exhibiting a yellow colour. As a result, plants which bloom one or more yellow coloured flowers can be produced. Plants of particular species cannot manage to bloom yellow coloured flowers in the natural state, and therefore, the polynucleotide of the present invention is very useful for producing plants, etc. which bloom flowers with a new colour that cannot exist in the natural state. Furthermore, the plant produced using the polynucleotide of the present invention can bloom flowers with a new colour that cannot exist in the natural state and is worthy for ornamental purposes, which is expected to have high commercial value. In addition, by using the polynucleotide of the present invention, hydroxylated flavonols at position 8 and hydroxylated flavanones and flavones can be produced.

### [Sequence listing free text]

SEQ ID NO: 4: synthetic DNA
SEQ ID NO: 5: synthetic DNA

## Claims

1. A polynucleotide according to any one selected from the group consisting of (a) to (e) below:
(a) a polynucleotide comprising the nucleotide sequence of SEQ ID NO: 1 or 3;
(b) a polynucleotide encoding a protein consisting of the amino acid sequence of SEQ ID NO: 2;
(c) a polynucleotide encoding a flavin enzyme protein consisting of an amino acid sequence wherein 1 to 100 amino acids are deleted, substituted, inserted and/or added in the amino acid sequence of SEQ ID NO: 2, and having a flavonol 8-hydroxylase activity;
(d) a polynucleotide encoding a flavin enzyme protein having an amino acid sequence having at least 70% identity with the amino acid sequence of SEQ ID NO: 2, and having a flavonol 8-hydroxylase activity; and,
(e) a polynucleotide that hybridizes to a polynucleotide consisting of a nucleotide sequence complementary to the nucleotide sequence of SEQ ID NO: 1 or 3 under stringent conditions, and that encodes a flavin enzyme protein having a flavonol 8-hydroxylase activity.

2. The polynucleotide according to claim 1, which is either one defined in (f) or (g) below:
(f) a polynucleotide encoding a flavin enzyme protein consisting of an amino acid sequence wherein 1 to 10 amino acids are deleted, substituted, inserted and/or added in the amino acid sequence of SEQ ID NO: 2, and having a flavonol 8-hydroxylase activity; and,
(g) a polynucleotide encoding a flavin enzyme protein having an amino acid sequence having at least 90% identity with the amino acid sequence of SEQ ID NO: 2, and having a flavonol 8-hydroxylase activity.

3. The polynucleotide according to claim 1, comprising the nucleotide sequence of SEQ ID NO: 1 or 3.

4. The polynucleotide according to claim 1, encoding a protein consisting of the amino acid sequence of SEQ ID NO: 2.

5. The polynucleotide according to any one of claims 1 to 4, which is a DNA.

6. A protein encoded by the polynucleotide according to any one of claims 1 to 5.

7. A vector comprising the polynucleotide according to any one of claims 1 to 5.

8. A non-human transformant, into which the polynucleotide according to any one of claims 1 to 5 is introduced.

9. A non-human transformant, into which the vector according to claim 7 is introduced.

10. The non-human transformant according to claim 8 or 9, which is a plant.

11. A method for screening a plant which blooms one or more yellow coloured flowers, which comprises the steps of:
(1) extracting a polynucleotide from a subject plant;
(2) hybridizing the polynucleotide to a polynucleotide that hybridizes to a polynucleotide consisting of a nucleotide sequence complementary to the nucleotide sequence of SEQ ID NO: 1 or 3 under stringent conditions; and,
(3) detecting the hybridization.

12. A method for producing a plant which blooms one or more yellow coloured flowers, said method comprising introducing the polynucleotide according to any one of claims 1 to 5 into a host plant or part thereof.

13. A method for producing a flavin enzyme protein having a flavonol 8-hydroxylase activity, said method comprising culturing the non-human transformant according to claim 8 or 9.

14. A processed product of the plant according to claim 10 or part thereof.

15. A method for producing a 8-hydroxylated flavonol, said method comprising reacting the protein according to claim 6 or a fragment of the protein having a flavonol 8-hydroxylase activity with a flavonol.
